(19) Europäisches Patentamt
European Patent Office
Office européen des brevets

(11) Veröffentlichungsnummer: **0 614 899 A1**

# (12) EUROPÄISCHE PATENTANMELDUNG

(21) Anmeldenummer: **94101885.5**

(22) Anmeldetag: **08.02.94**

(51) Int. Cl.[5]: **C07D 498/06**, C07D 513/06, C07D 279/16, C07D 265/36, A61K 31/535, A61K 31/54, //(C07D498/06,265:00,209:00), (C07D513/06,279:00,209:00)

(30) Priorität: **17.02.93 DE 4304806**

(43) Veröffentlichungstag der Anmeldung: **14.09.94 Patentblatt 94/37**

(84) Benannte Vertragsstaaten: **BE CH DE ES FR GB IT LI NL**

(71) Anmelder: **Kali-Chemie Pharma GmbH**
**Hans-Böckler-Allee 20**
**D-30173 Hannover (DE)**

(72) Erfinder: **Demonchaux, Patrice**
**27, Avenue Clément Désormes**
**F-01400 Châtillon sur Chalaronne (FR)**
Erfinder: **Lenoir, Patrick**
**Les Bruyères**
**F-01400 Romans (FR)**

(74) Vertreter: **Lauer, Dieter, Dr.**
**c/o Solvay Deutschland GmbH**
**Hans-Böckler-Allee 20**
**D-30173 Hannover (DE)**

(54) **5-Phenyl-pyrrolo-1,4-benzoxazin- und -thiazin-Derivate sowie Verfahren und Zwischenprodukte zu ihrer Herstellung und diese Verbindungen enthaltende Arzneimittel.**

(57) Es werden pharmakologisch wirksame Verbindungen der allgemeinen Formel I

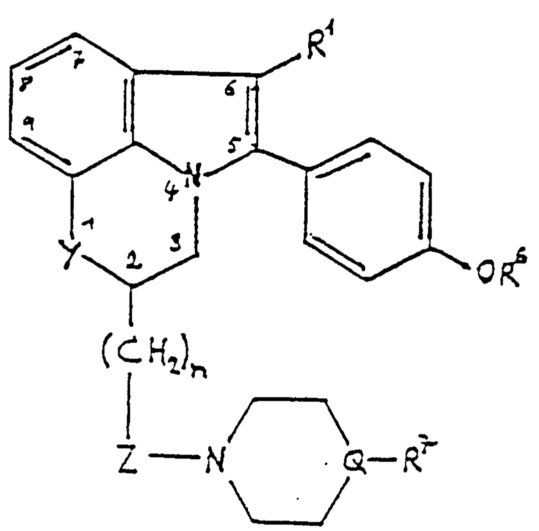

I

welche in den Phenylringen substituiert sein können und worin

$R^1$ Wasserstoff oder niederes Alkyl bedeutet,
$R^6$ Wasserstoff oder niederes Alkyl bedeutet,

EP 0 614 899 A1

Y Sauerstoff oder Schwefel bedeutet,
n für eine Zahl von 1 bis 3 steht,
Z eine Bindung, die CO-Gruppe oder die $CH_2$-Gruppe darstellt,
Q Stickstoff oder die CH-Gruppe bedeutet und
$R^7$ ,falls Q Stickstoff bedeutet, für einen gegebenenfalls substituierten Pyridyl- oder Phenylrest steht, oder, falls Q die CH-Gruppe bedeutet, für die N-Methyl-N-(4-oxo-3H-pyrimidin-2-yl)-aminogruppe steht,

und deren Säureadditionssalze sowie Verfahren und Zwischenprodukte zu ihrer Herstellung beschrieben.

Die vorliegende Erfindung betrifft neue 5-Phenyl-pyrrolo-1,4-benzoxazin- und -thiazin-Derivate, welche in 2-Stellung des Ringgerüstes einen substituierten Piperazinoalkyl- oder Piperidinoalkylrest tragen, und deren Salze sowie diese Verbindungen enthaltende pharmazeutische Zubereitungen und Verfahren und Zwischenprodukte zur Herstellung dieser Verbindungen.

Aus der EP 0 322 016 A1 sind Ester und Amide von 1,7-anellierten Indol-2-carbonsäure-derivaten und cyclischen Alkoholen oder Aminen bekannt, welche selektive Antagonisten von neuronalen 5-HT-Rezeptoren darstellen und zur Behandlung von durch Überstimmulation dieser Rezeptoren induzierten Beschwerden, beispielsweise im gastrointestinalen Bereich, geeignet sind.

Aus der EP 0 387 618 A1 sind Amide aus 1,7-anellierten Indol-2-carbonsäurederivaten mit 3-Amino-1,4-benzodiazepin-derivaten bekannt. Diese Verbindungen besitzen CCK-antagonistische Wirkungen mit einer die Magenentleerung fördernden Wirkungskomponente.

Der vorliegenden Erfindung liegt die Aufgabe zugrunde, neue als Antiallergika einsetzbare pharmazeutische Wirkstoffe zu entwickeln. Ferner liegt der Erfindung die Aufgabe zugrunde, neue Derivate von 1,7-anellierten Indol-Verbindungen mit wertvollen pharmakologischen Eigenschaften herzustellen.

Es wurde nun gefunden, daß die erfindungsgemäßen neuen 5-Phenyl-pyrrolo-1,4-benzoxazin- und -thiazin-derivate wertvolle pharmakologische Eigenschaften besitzen und antiinflammatorische und antiallergische Wirkungen zeigen und ein günstiges Wirkungsprofil mit geringer Toxizität und guter Verträglichkeit aufweisen. Aufgrund ihres Wirkungsprofils eignen sich die erfindungsgemäßen Substanzen als antiinflammatorisch wirksame Wirkstoffe und Antiallergika zur Behandlung von entzündlichen oder allergischen Erkrankungen.

Die vorliegende Erfindung betrifft daher neue Verbindungen der allgemeinen Formel I
(siehe Formel I)

worin

- $R^1$ Wasserstoff oder niederes Alkyl bedeutet,
- $R^2$ Wasserstoff, Halogen, niederes Alkyl, niederes Alkoxy, Benzyloxy oder Hydroxy bedeutet,
- $R^3$ Wasserstoff, Halogen, niederes Alkyl, niederes Alkoxy oder Hydroxy bedeutet,
- $R^4$ Wasserstoff, niederes Alkyl, niederes Alkoxy oder Hydroxy bedeutet,
- $R^5$ Wasserstoff, niederes Alkyl, niederes Alkoxy oder Hydroxy bedeutet,
- $R^6$ Wasserstoff oder niederes Alkyl bedeutet,
- Y Sauerstoff oder Schwefel bedeutet,
- n für eine Zahl von 1 bis 3 steht,
- Z eine Bindung, die CO-Gruppe oder die $CH_2$-Gruppe darstellt,
- Q Stickstoff oder die CH-Gruppe bedeutet und
- $R^7$ ,falls Q Stickstoff bedeutet, für einen gegebenenfalls durch niederes Alkyl oder Halogen substituierten Pyridyl- oder Phenylrest steht, oder, falls Q die CH-Gruppe bedeutet, für die N-Methyl-N-(4-oxo-3H-pyrimidin-2-yl)-aminogruppe steht,

und deren physiologisch verträgliche Säureadditionssalze.

Sofern in den Verbindungen der Formel I die Substituenten niedere Alkylgruppen bedeuten oder enthalten, können diese Alkylgruppen gerade oder verzweigt sein und insbesondere 1 bis 4, vorzugsweise 1 bis 2 Kohlenstoffatome enthalten und stellen bevorzugt Methyl dar. Sofern die Substituenten Halogen bedeuten oder Halogensubstituenten enthalten, kommen insbesondere Fluor, Chlor oder Brom, vorzugsweise Fluor oder Chlor in Frage.

Der Substituent $R^1$ kann Wasserstoff oder niederes Alkyl bedeuten und steht vorzugsweise für niederes Alkyl, insbesondere Methyl. Der Benzolring des tricyclischen Ringgerüstes ist vorzugsweise unsubstituiert oder in 7- oder 8-Stellung monosubstituiert. Als Substituenten eignen sich insbesondere niederes Alkoxy, vorzugsweise Methoxy, Hydroxy oder auch Halogen, vorzugsweise Chlor oder Fluor. Der 5-Phenylsubstituent trägt vorzugsweise eine freie Hydroxygruppe in 4-Stellung. Dementsprechend steht $R^6$ vorzugsweise für Wasserstoff. Falls $R^6$ niederes Alkyl bedeutet, stellt dieses vorzugsweise Methyl dar. Das Ringglied Y stellt vorzugsweise Sauerstoff dar.

Die $(CH_2)_n$-Z-Kette stellt vorzugsweise eine Kette mit 2 bis 3 Kohlenstoffatomen, insbesondere die Ethylenkette oder eine Propylen- oder Oxopropylenkette dar.

Falls der Substituent $R^7$ einen Pyridylrest darstellt, kann dieser unsubstituiert oder durch niederes Alkyl oder Halogen substituiert sein. Beispielsweise eignen sich durch niederes Alkyl, insbesondere Methyl, substituierte oder unsubstituierte Pyridylreste. Bevorzugt kommt eine Pyridin-2-yl-Gruppe in Frage, welche gegebenenfalls substituiert sein kann. Als besonders günstig erweist sich der 4-Methylpyridin-2-yl-rest.

Die Verbindungen der Formel I enthalten ein chirales Kohlenstoffatom in 2-Stellung des Ringgerüstes und können in mehreren optisch aktiven enantiomeren Formen oder als Racemate vorliegen. Die vorliegende Erfindung umfaßt sowohl die racemischen Gemische wie auch die reinen optischen Isomeren der

Verbindungen der Formel I.

Erfindungsgemäß werden die neuen Verbindungen der Formel I und deren Säureadditionssalze erhalten, indem man in an sich bekannter Weise

a) zur Herstellung von Verbindungen der allgemeinen Formel Ia

(siehe Formel Ia)

worin $R^1$, $R^2$, $R^3$, $R^4$, $R^5$, $R^6$, n, Y, Q und $R^7$ obige Bedeutung besitzen und Z' eine Bindung oder die $CH_2$-Gruppe darstellt, Verbindungen der allgemeinen Formel IIa

(siehe Formel IIa)

worin $R^1$, $R^2$, $R^3$, $R^4$, $R^5$, $R^6$, n, Z' und Y obige Bedeutung besitzen, und X für eine aminolytisch abspaltbare Fluchtgruppe steht,

mit Verbindungen der allgemeinen Formel III

(siehe Formel III)

worin Q und $R^7$ obige Bedeutung besitzen, umsetzt oder

b) zur Herstellung von Verbindungen der allgemeinen Formel Ib

(siehe Formel Ib)

worin $R^2$, $R^3$, $R^4$, $R^5$, $R^6$, Y, n, Q und $R^7$ obige Bedeutung besitzen und $R^{1'}$ niederes Alkyl bedeutet, Verbindungen der allgemeinen Formel IVa

(siehe Formel IVa)

worin $R^{1'}$, $R^2$, $R^3$, $R^4$, $R^5$, $R^6$, Y und n obige Bedeutung besitzen,

mit Verbindungen der Formel III umsetzt oder

c) zur Herstellung von Verbindungen der allgemeinen Formel Ic

(siehe Formel Ic)

worin $R^1$, $R^2$, $R^3$, $R^4$, $R^5$, $R^6$, Y und n obige Bedeutung besitzen und $R^{7'}$ für einen gegebenenfalls durch niederes Alkyl oder Halogen substituierten Pyridyl- oder Phenylrest steht, Verbindungen der allgemeinen Formel Id

(siehe Formel Id)

worin $R^1$, $R^2$, $R^3$, $R^4$, $R^5$, $R^6$, Y, n und $R^{7'}$ obige Bedeutung besitzen, reduziert, oder

d) zur Herstellung von Verbindungen der allgemeinen Formel Ie

(siehe Formel Ie)

worin $R^2$, $R^3$, $R^4$, $R^5$, $R^6$, Y, n, Z, Q und $R^7$ obige Bedeutung besitzen, aus Verbindungen der allgemeinen Formel V

(siehe Formel V)

worin $R^2$, $R^3$, $R^4$, $R^5$, $R^6$, Y, n, Z, Q und $R^7$ obige Bedeutung besitzen und $R^8$ niederes Alkyl bedeutet, die $COOR^8$-Gruppe abspaltet, oder

e) zur Herstellung von Verbindungen der allgemeinen Formel If

(siehe Formel If)

worin $R^{1'}$, $R^2$, $R^3$, $R^4$, $R^5$, $R^6$, Y, n, Z, Q und $R^{7'}$ obige Bedeutung besitzen, Verbindungen der allgemeinen Formel VI

(siehe Formel VI)

worin $R^2$, $R^3$, Y, n, Z und $R^{7'}$ obige Bedeutung besitzen, umsetzt mit Verbindungen der allgemeinen Formel VIIa

(siehe Formel VIIa)

worin $R^{1'}$, $R^4$, $R^5$ und $R^6$ obige Bedeutung besitzen,

und in den erhaltenen Verbindungen der Formel I gewünschtenfalls Methoxysubstituenten $R^2$, $R^3$, $R^4$, $R^5$ und/oder $OR^6$ in Hydroxy überführt und/oder einen Benzyloxysubstituenten $R^2$ in Hydroxy überführt,

und die erhaltenen Verbindungen der Formel I gewünschtenfalls in ihre Säureadditionssalze überführt oder die Säureadditionssalze in die freien Verbindungen der Formel I überführt.

Bei den nachfolgend beschriebenen Reaktionen ist es im allgemeinen zweckmäßig, freie phenolische Hydroxygruppen in den Ausgangsverbindungen während den Reaktionen durch leicht wieder abspaltbare Schutzgruppen zu schützen und diese Schutzgruppen anschließend wieder abzuspalten. Als Schutzgruppen können an sich bekannte Schutzgruppen gewählt werden, welche anschließend auf an sich bekannte Weise solvolytisch oder hydrogenolytisch wieder abgespalten werden können. Geeignete leicht wieder abspaltbare Schutzgruppen für phenolische OH-Gruppen sind zum Beispiel bekannt aus E. McOmie "Protective Groups in Organic Chemistry" Plenum Press 1971. Als Schutzgruppen für allfällige phenolische Hydroxygruppen können beispielsweise an sich bekannte Etherschutzgruppen gewählt werden, zum Beispiel niedere Alkylgruppen oder gegebenenfalls substituierte Benzylgruppen. Schutzgruppen müssen selbstverständlich unter Berücksichtigung der übrigen in der zu schützenden Verbindung enthaltenden Reste jeweils so ausgewählt werden, daß sie anschließend leicht unter Bedingungen, unter denen andere im Molekül

enthaltene Reste nicht angegriffen werden, abspaltbar sind.

Die Umsetzung von Verbindungen der Formel IIa mit Verbindungen der Formel III gemäß Verfahrensvariante a) kann nach an sich zur Alkylierung von Aminen üblichen Methoden ausgeführt werden. Die Umsetzung wird zweckmäßigerweise in einem unter den Reaktionsbedingungen inerten organischen Lösungsmittel unter basischen Bedingungen durchgeführt.

Als aminolytisch abspaltbare Reste in den Verbindungen der Formel IIa eignen sich Halogene wie Chlor, Brom oder Jod oder ein Acyloxyrest O-E, worin E einen niederen Alkanoylrest oder einen organischen Sulfonsäurerest darstellt, beispielsweise den Rest von einer Niederalkansulfonsäure, wie zum Beispiel Methansulfonsäure, oder von aromatischen Sulfonsäuren, wie Benzolsulfonsäure oder durch niederes Alkyl oder durch Halogen substituierten Benzolsulfonsäuren wie Brombenzolsulfonsäuren. Als besonders geeignet erweist sich zum Beispiel ein Toluolsulfonsäurerest. Als inerte organische Lösungsmittel eignen sich insbesondere aprotische Lösungsmittel, vorzugsweise Dimethylformamid und beispielsweise auch aromatische Kohlenwasserstoffe wie Toluol, Xylol oder Benzol, cyclische Ether wie Dioxan, niedere Alkanole wie Ethanol oder Gemische aus den vorgenannten Lösungsmitteln. Zweckmäßigerweise wird bei erhöhten Temperaturen, beispielsweise Temperaturen zwischen 50 und 120 °C, gearbeitet. Zweckmäßigerweise wird die Reaktion unter Zusatz einer zum Abfangen der sich bildenden Säure ausreichenden Menge einer organischen oder anorganischen Base durchgeführt. Es kann jedoch auch ein Überschuß der Verbindung der Formel III verwendet und dieser als interne Base benutzt werden. Beispiele geeigneter organischer Basen sind tertiäre organische Amine, insbesondere tertiäre niedere Alkylamine wie Triethylamin, Tripropylamine, N-Niederalkylmorpholine oder N-Niederalkylpiperazine. Geeignete anorganische Basen sind insbesondere Alkalimetallcarbonate oder -bicarbonate.

Die Umsetzung von Säuren der Formel IVa mit Aminen der Formel III kann nach an sich zur Bildung von Amidgruppierungen durch Aminoacylierung üblichen Methoden ausgeführt werden. So kann die Umsetzung einer Aminoverbindung der Formel III mit einer Säure der Formel IVa zweckmäßig in Gegenwart eines aus der Peptidchemie als zur Amidbildung geeignet bekannten Kopplungsreagenzes durchgeführt werden. Als Beispiele von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt Carbonyldiimidazol oder Alkylcarbodiimide, zum Beispiel Cycloalkylcarbodiimide wie Dicyclohexylcarbodiimid, und N-Niederalkyl-2-halogenpyridiniumsalze, insbesondere Halogenide oder Tosylate, beispielsweise N-Methyl-2-chlorpyridiniumjodid (siehe zum Beispiel Mukajama in Angew. Chemie 91 789 bis 812), oder auch Chlorameisensäureniederalkylester. Die Umsetzung in Gegenwart eines Kopplungsreagenzes kann zweckmäßig bei Temperaturen von -30 °C bis +50 °C, vorzugsweise bei annähernd Raumtemperatur, in nicht polaren, aprotischen organischen Lösungsmitteln wie halogenierten Kohlenwasserstoffen, beispielsweise Dichlormethan und/oder cyclischen Ethern wie Tetrahydrofuran und/oder aromatischen Lösungsmitteln, gegebenenfalls in Gegenwart eines säurebindenden Amins durchgeführt werden. Gewünschtenfalls können die Säuren der Formel IVa auch zunächst auf an sich bekannte Weise in reaktionsfähige Säurederivate, insbesondere Säurehalogenide, gemischte Säureanhydride oder Ester überführt werden und diese dann auf an sich bekannte Weise mit den Verbindungen der Formel III umgesetzt werden.

Die Reduktion von Amidverbindungen der Formel Id gemäß Verfahrensvariante c) kann nach an sich zur Reduktion von Amiden üblichen Methoden erfolgen. Als Reduktionsmittel eignen sich zur Amidreduktion befähigte komplexe Metallhydride, insbesondere Borankomplexe, beispielsweise ein Boran/Tetrahydrofuran-Komplex in Tetrahydrofuran, oder auch Aluminiumhydride wie Lithiumaluminiumhydrid oder Natrium-bis(2-methoxyethoxy)-aluminiumhydrid oder Lithiumborhydrid. Die Reaktion findet in einem unter den Reaktionsbedingungen inerten ausreichend wasserfreien organischen Lösungsmittel statt. Als Lösungsmittel eignen sich beispielsweise cyclische Ether wie Tetrahydrofuran oder Dioxan oder offenkettige Ether wie Diethylether, Ethylenglycoldimethylether oder Diethylenglycoldimethylether, gegebenenfalls im Gemisch mit aromatischen Kohlenwasserstoffen wie Benzol oder Toluol. Die Reaktionstemperatur kann je nach Art des verwendeten Reduktionsmittels zwischen 0 °C und Siedetemperatur des Reaktionsgemisches variieren. Als günstig erweist sich beispielsweise die Reduktion mit einem Boran/Tetrahydrofuran-Komplex in Tetrahydrofuran bei Temperaturen zwischen Raumtemperatur und Siedetemperatur des Reaktionsgemisches.

Die Abspaltung der $COOR^8$-Gruppe aus Verbindungen der allgemeinen Formel V gemäß Verfahrensvariante d) kann auf an sich bekannte Weise erfolgen, indem die $COOR^8$-Gruppe zunächst unter basischen Bedingungen hydrolysiert wird und das Hydrolyseprodukt anschließend decarboxyliert wird. Die Hydrolyse der $COOR^8$-Gruppe erfolgt zweckmäßig durch Behandeln der Verbindung der Formel V mit einer anorganischen Base, beispielsweise einem Alkalimetallhydroxid, in einem protischen Lösungsmittel, vorzugsweise einem niederen Alkohol wie beispielsweise Ethanol oder einem Alkoholwassergemisch. Die Reaktionstemperatur für die Hydrolyse kann zwischen Raumtemperatur und Siedepunkt des Lösungsmittels betragen.

Die erhaltene Säure wird anschließend durch Erhitzen auf Temperaturen zwischen 150 und 200 °C decarboxyliert. Die Decarboxylierung kann ohne Lösungsmittel oder in einem entsprechend hochsiedenden Lösungsmittel durchgeführt werden. Die Reaktionsdauer kann zwischen 1 und 12 Stunden betragen. Eine allfällige Amidgruppierung in der Seitenkette in Position 2 des tricyclischen Ringgerüstes wird unter den vorgenannten Bedingungen einer basischen Hydrolyse und anschließenden Decarboxylierung nicht angegriffen.

Die Umsetzung der Hydrazin-Verbindungen der Formel VI mit den Carbonylverbindungen der Formel VIIa gemäß Verfahrensvariante e) kann auf an sich bekannte Weise erfolgen, indem man die Verbindungen beispielsweise miteinander unter den Bedingungen einer Fischerschen Indolsynthese umsetzt. Die Umsetzung kann durch Erhitzen auf Temperaturen zwischen 50 und 100 °C in saurem Medium erfolgen. Zum Beispiel kann die Reaktion in einem mit Wasser mischbaren säurehaltigen organischen Lösungsmittel, beispielsweise einem mit wäßriger Salzsäure, Bromwasserstoffsäure, Schwefelsäure oder Phosphorsäure oder auch einer organischen Säure, zum Beispiel einer organischen Sulfonsäure wie einer Toluolsulfonsäure, angesäuerten organischen Lösungsmittel wie einem niederen Alkohol oder Essigsäure durchgeführt werden. Die Reaktionstemperatur kann zwischen ca. 25 und 90 °C betragen.

In Verbindungen der Formel I, worin die $R^6O$-Gruppe und/oder die Substituenten $R^2$, $R^3$, $R^4$ und/oder $R^5$ Methoxy darstellen, können die Methoxygruppen mit zur Spaltung von Methoxyarylethern geeigneten Methoden auf an sich bekannte Weise zu Hydroxygruppen gespalten werden. Beispielsweise kann die Etherspaltung durch Behandeln mit Bortribromid in einem sauerstofffreien organischen Lösungsmittel, insbesondere einem halogeniertem Kohlenwasserstoff wie Dichlormethan oder einem niedrig siedenden aliphatischen Kohlenwasserstoff wie n-Pentan erfolgen. Die Demethylierung kann auch durch Behandeln mit anderen Demethylierungsmitteln wie zum Beispiel Jodtrimethylsilan, Lewissäuren wie beispielsweise Aluminiumtrichlorid, oder einem Bortrifluoriddimethylthioetherkomplex in einem halogeniertem Kohlenwasserstoff wie Dichlormethan oder Chloroform durchgeführt werden. Eine Benzyloxygruppe $R^2$ kann gewünschtenfalls auf an sich bekannte Weise, z. B. durch katalytische Hydrogenolyse in eine Hydroxygruppe überführt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, zum Beispiel Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Maleinsäure, Fumarsäure, Milchsäure, Weinsäure oder Essigsäure, oder Sulfonsäuren, beispielsweise Niederalkansulfonsäure wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Falls bei der Synthese der Verbindungen der Formel I Racemate der Ausgangsverbindungen der Formeln IIa, IVa, V oder VI eingesetzt werden, werden die Verbindungen der Formel I in Form von Racematen erhalten. Ausgehend von optisch aktiven Formen der Ausgangsverbindungen können optisch aktive Verbindungen der Formel I erhalten werden. Die optisch aktiven Verbindungen der Formel I können aus den racemischen Gemischen in an sich bekannter Weise erhalten werden, zum Beispiel durch chromatographische Trennung an chiralen Trennmaterialien oder im Falle von Verbindungen der Formel I, worin Z eine Bindung oder die $CH_2$-Gruppe bedeutet, durch Umsetzung mit geeigneten optisch aktiven Säuren, beispielsweise Weinsäure, Mandelsäure oder 10-Campher-sulfonsäure, und anschließende Auftrennung in ihre optisch aktiven Antipoden durch fraktionierte Kristallistion der gewonnenen Salze. Diese können dann wiederum in die freien Basen überführt werden. Falls Z für eine CO-Gruppe steht, ist es zweckmäßig für die Herstellung von optisch aktiven Verbindungen der Formel I bereits von optisch aktiven Zwischenprodukten auszugehen. Die als Zwischenprodukte dienenden optisch aktiven Säurederivate, beispielsweise die Verbindungen der Formel IV, können aus den racemischen Gemischen dieser Säurederivate in an sich bekannter Weise erhalten werden, indem man durch Umsetzung mit geeigneten optisch aktiven Alkoholen, beispielsweise Menthol oder Octanol, oder optisch aktiven Aminen, beispielsweise 1-(1-Naphtyl)ethylamin oder 2-Methylbenzylamin, entsprechende diastereoisomere Ester, Amide oder Ammoniumsalze herstellt, diese auf an sich bekannte Weise durch Chromatographie trennt und die erhaltenen Enantiomeren wieder in die freien Säuren überführt.

Die Ausgangsverbindungen der Formel II

(siehe Formel II)

worin $R^2$, $R^3$, $R^4$, $R^5$, $R^6$, Y, n, Z' und X obige Bedeutung besitzen und $R^{1''}$ die für $R^1$ angegebene

Bedeutung besitzt oder die niedere Alkoxycarbonylgruppe $COOR^8$, worin $R^8$ obige Bedeutung besitzt, darstellt, sind neue Verbindungen, welche wertvolle Zwischenprodukte zur Herstellung von pharmakologisch wirksamen Verbindungen, beispielsweise den Verbindungen der Formel I, darstellen.

Die Verbindungen der Formel II können auf an sich bekannte Weise ausgehend von den entsprechenden Alkoholen der allgemeinen Formel VIII

(siehe Formel VIII)

worin $R^{1''}$, $R^2$, $R^3$, $R^4$, $R^5$, $R^6$, Y, n und Z' obige Bedeutung besitzen, erhalten werden, indem die Hydroxygruppe in an sich bekannter Weise in eine Fluchtgruppe X überführt wird. So können die Verbindungen der Formel VIII beispielsweise zur Einführung eines Halogenrestes X mit Thionylchlorid oder mit Phosphorsäurehalogeniden, zum Beispiel Phosphortribromid, auf an sich bekannte Weise in einem unter den Reaktionsbedingungen inerten Lösungsmittel, zum Beispiel einem halogeniertem Kohlenwasserstoff wie Chloroform, umgesetzt werden. Sulfonsäurereste X können auf an sich bekannte Weise eingeführt werden, indem man Verbindungen der Formel VIII mit einem entsprechenden Sulfonsäurechlorid, vorzugsweise p-Toluolsulfonsäurechlorid, acylliert.

Alkohole der Formel VIII sind neue Verbindungen, welche wertvolle Zwischenprodukte zur Herstellung von pharmakologisch aktiven Verbindungen darstellen. Alkohole der allgemeinen Formel VIIIa

(siehe Formel VIIIa)

worin $R^2$, $R^3$, $R^4$, $R^5$, $R^6$, Y, n und Z' obige Bedeutung besitzen und $R^{1'''}$ niederes Alkyl oder eine $COOR^8$-Gruppe bedeutet, können auf an sich bekannte Weise erhalten werden, indem man Hydrazinverbindungen der allgemeinen Formel IX

(siehe Formel IX)

worin $R^2$, $R^3$, Y, n und Z' obige Bedeutung besitzen, mit Carbonylverbindungen der allgemeinen Formel VII

(siehe Formel VII)

worin $R^{1'''}$, $R^4$, $R^5$ und $R^6$ obige Bedeutung besitzen, auf an sich bekannte Weise kondensiert. Die Umsetzung der Verbindungen der Formel IX mit den Verbindungen der Formel VII kann beispielsweise unter den vorstehend für die Verfahrensvariante e) zur Umsetzung von Verbindungen der Formel VI mit Verbindungen der Formel VIIa angegebenen Reaktionsbedingungen durchgeführt werden.

Zur Herstellung von Verbindungen der Formel VIII, worin $R^{1''}$ Wasserstoff bedeutet, kann eine allfällige $COOR^8$-Gruppe aus Verbindungen der Formel VIIIa auf an sich bekannte Weise abgespalten werden. Die Abspaltung kann beispielsweise in der für die Herstellung von Verbindungen der Formel Ie gemäß Verfahrensvariante d) beschriebenen Weise erfolgen.

Alkohole der allgemeinen Formel VIIIb

(siehe Formel VIIIb)

worin $R^{1'}$, $R^2$, $R^3$, $R^4$, $R^5$, $R^6$, Y und n obige Bedeutung besitzen, können auch ausgehend von entsprechenden Nitrilverbindungen der allgemeinen Formel X

(siehe Formel X)

worin $R^{1'}$, $R^2$, $R^3$, $R^4$, $R^5$, $R^6$, Y und n obige Bedeutung besitzen, erhalten werden, indem man die CN-Gruppe auf an sich bekannte Weise in eine $CH_2OH$-Gruppe überführt. Hierzu werden die Nitrilverbindungen der Formel X zunächst auf an sich bekannte Weise durch saure Solvolyse in einem niederen Alkohol in entsprechende Ester der allgemeinen Formel XXa

(siehe Formel XXa)

worin $R^{1'}$, $R^2$, $R^3$, $R^4$, $R^5$, $R^6$, Y und n obige Bedeutung besitzen und $R^9$ niederes Alkyl bedeutet, überführt. Hierzu kann eine Lösung der Verbindung der Formel X in einem niederen Alkohol mit Säure, beispielsweise einer Halogenwasserstoffsäure, welche zweckmäßigerweise als Halogenwasserstoff-Gas in die Lösung eingeleitet wird, behandelt werden.

Die so erhaltenen Verbindungen der Formel XXa können anschließend auf an sich bekannte Weise reduziert werden. Als Reduktionsmittel eignen sich beispielsweise zur Reduktion von Estern zu Alkoholen befähigte Hydridreduktionsmittel, beispielsweise die vorstehend unter Verfahrensvariante c) zur Reduktion von Verbindungen der Formel Id angegebenen Reduktionsmittel, insbesondere Lithiumaluminiumhydrid oder Natrium-bis(2-methoxyethoxy)aluminiumhydrid. Die Reduktion kann nach üblichen Methoden, beispielsweise bei den für die Reduktion von Verbindungen der Formel Id gemäß Verfahrensvariante c) angegebenen Reaktionsbedingungen, durchgeführt werden. Als zweckmäßig erweist sich insbesondere die Reduktion mit Lithiumaluminiumhydrid in Tetrahydrofuran.

Verbindungen der Formel IX können auf an sich bekannte Weise, ausgehend von Verbindungen der allgemeinen Formel XI

(siehe Formel XI)

worin $R^2$, $R^3$, Y, n und Z' obige Bedeutung besitzen, erhalten werden. Hierzu werden die Verbindungen der Formel XI durch Behandeln mit Natriumnitrit auf an sich bekannte Weise in die entsprechenden N-Nitrosoverbindungen der allgemeinen Formel XII

(siehe Formel XII)

worin $R^2$, $R^3$, Y, n und Z' obige Bedeutung besitzen, überführt und diese anschließend zu den Hydrazinverbindungen der Formel XI reduziert. Für die Reduktion der Nitrosoverbindungen der Formel XII können alle an sich zur Reduktion von Nitrosoverbindungen zu entsprechenden Hydrazinverbindungen bekannten Reduktionsmethoden eingesetzt werden. Als Reduktionsmittel eignen sich beispielsweise Lithiumaluminiumhydrid in Tetrahydrofuran oder metallisches Zinkpulver in Gegenwart einer Säure oder Natriumdithionit. Auch eine katalytische Hydrierung der Nitrosoverbindungen zu den Hydrazinen der Formel IX ist möglich. Verbindungen der Formel IX können auch, ausgehend von Ester-Verbindungen der allgemeinen Formel XIII

(siehe Formel XIII)

worin $R^2$, $R^3$, Y und $R^9$ obige Bedeutung besitzen und n' für 0 bis 3 steht, erhalten werden, indem man diese zunächst auf an sich bekannte Weise durch Behandeln mit Natriumnitrit in die entsprechenden N-Nitrosoverbindungen der allgemeinen Formel XXX

(siehe Formel XXX)

worin $R^2$, $R^3$, Y , n' und $R^9$ obige Bedeutung besitzen, überführt und diese anschließend zu den Hydrazinverbindungen der Formel IX reduziert. Für die Reduktion der Nitrosoverbindungen der Formel XXX werden Reduktionsmittel eingesetzt, welche sowohl die Nitrosofunktion wie auch die Carbonylfunktion reduzieren können. Als geeignet erweist sich insbesondere Lithiumaluminiumhydrid. In den Verbindungen der Formel XXX kann zunächst auch nur die Nitrosofunktion mit einem die Carbonylfunktion nicht angreifenden Reduktionsmittel, beispielsweise mit metallischen Zinkpulver in Gegenwart von Säure, reduziert werden und in den so erhaltenen Verbindungen der allgemeinen Formel XXI

(siehe Formel XXI)

worin $R^2$, $R^3$, Y, n' und $R^9$ obige Bedeutung besitzen, anschließend die Carbonylfunktion auf an sich bekannte Weise, beispielsweise mit Lithiumaluminiumhydrid, zur $CH_2OH$-Gruppe reduziert.

Alkohole der Formel XI können erhalten werden, indem man Ester-Verbindungen der Formel XIII auf an sich bekannte Weise reduziert. Die Reduktion kann beispielsweise unter den vorstehend für die Reduktion der Ester-Verbindungen der Formel XXa zu den Alkoholen der Formel VIIIb angegebenen Bedingungen durchgeführt werden.

Alkohol-Verbindungen der allgemeinen Formel XIa

(siehe Formel XIa)

worin $R^2$, $R^3$, Y und n obige Bedeutung besitzen, können auch durch Reduktion von Verbindungen der allgemeinen Formel XIV

(siehe Formel XIV)

worin $R^2$, $R^3$, Y, n und $R^9$ obige Bedeutung besitzen, erhalten werden. Die Reduktion kann auf an sich bekannte Weise mit zur Reduktion von Estern zu entsprechenden Alkoholen geeigneten Reduktionsmittel erfolgen. Beispielsweise kann die Reduktion mit Lithiumaluminiumhydrid in einem cyclischen Ether wie Tetrahydrofuran bei erhöhter Temperatur, vorzugsweise Siedetemperatur des Reaktionsgemisches, durchgeführt werden. Bei diesen Bedingungen werden sowohl die Estergruppe wie die Oxogruppe des Ringgerüstes reduziert.

Ester-Verbindungen der allgemeinen Formel XIIIa

(siehe Formel XIIIa)

worin $R^2$, $R^3$, Y, n und $R^9$ obige Bedeutung besitzen, können aus Verbindungen der Formel XIV durch selektive Reduktion der Oxogruppe des Ringgerüstes erhalten werden. Als selektive Reduktionsmittel eignen sich beispielsweise Diboran oder ein Borhydrid/Tetrahydrofuran-Komplex in Tetrahydrofuran.

Verbindungen der allgemeinen Formel XIIIb

(siehe Formel XIIIb)

worin $R^2$, $R^3$, Y und $R^9$ obige Bedeutung besitzen, können ausgehend von 2-Aminophenolen oder -thiophenolen der allgemeinen Formel XV

(siehe Formel XV)

worin $R^2$, $R^3$ und Y obige Bedeutung besitzen, erhalten werden, indem man diese mit einem 2,3-Dibrompropionsäureniederalkylester der allgemeinen Formel XVI

(siehe Formel XVI)

worin $R^9$ obige Bedeutung besitzt, umsetzt. Die Umsetzung kann auf an sich bekannte Weise in einem unter den Reaktionsbedingungen inerten organischen Lösungsmittel, beispielsweise Dimethylformamid erfolgen. Die Umsetzung wird zweckmäßigerweise unter Zusatz einer zum Abfangen der freigesetzten Säure ausreichende Menge einer Base, beispielsweise einer anorganischen Base wie einem Alkalimetallcarbonat durchgeführt. Die Reaktionstemperatur kann zwischen Raumtemperatur und 60 °C liegen.

Verbindungen der Formel XIV können ausgehend von 2-Nitrophenolen bzw. Nitrothiophenolen der allgemeinen Formel XVII

(siehe Formel XVII)

worin $R^2$, $R^3$ und Y obige Bedeutung besitzen, erhalten werden, indem man diese zunächst mit Verbindungen der allgemeinen Formel XVIII

(siehe Formel XVIII)

worin n und $R^9$ obige Bedeutung besitzen, umsetzt zu Verbindungen der allgemeinen Formel XIX

(siehe Formel XIX)

worin $R^2$, $R^3$, Y, n und $R^9$ obige Bedeutung besitzen, und diese anschließend unter hydrierenden Bedingungen cyclisiert. Die Umsetzung der Verbindungen der Formel XVII mit den Verbindungen der Formel XVIII kann auf an sich bekannte Weise unter zur Phenoletherbildung üblichen Bedingungen erfolgen. Die anschließende Cyclisierung der Verbindungen der Formel XIX kann auf an sich bekannte Weise unter den Bedingungen einer katalytischen Hydrierung erfolgen. So können die Verbindungen in einem unter den Reaktionsbedingungen inerten organischen Lösungsmittel, vorzugsweise einem niederen Alkohol mit Wasserstoff in Gegenwart eines Hydrierungskatalysators behandelt werden. Als Hydrierungskatalysatoren eignen sich Palladiumkatalysatoren, insbesondere Palladium/Kohle, oder auch Platin- oder Rhodiumkatalysatoren. Letztere erweisen sich als zweckmäßig für den Fall, daß die Substituenten $R^{2'}$ und/oder $R^3$ Halogen bedeuten.

Verbindungen der allgemeinen Formel XIVa

(siehe Formel XIVa)

worin $R^2$, $R^3$, Y und $R^9$ obige Bedeutung besitzen, können auch ausgehend von Aminophenolen bzw. Aminothiophenolen der Formel XV erhalten werden, indem diese mit Maleinsäureanhydrid in einem niederen Alkohol $R^9OH$ umgesetzt werden. Die Umsetzung kann in Gegenwart einer organischen Base, beispielsweise einem tertiären Niederalkylamin, bei erhöhter Temperatur, vorzugsweise Siedetemperatur des Reaktionsgemisches durchgeführt werden.

Die Aminophenole und Aminothiophenole der Formel XV sind bekannt oder können auf an sich bekannte Weise erhalten werden, beispielsweise durch Reduktion entsprechender Nitrophenole oder Nitrothiophenole der Formel XVII. Die Verbindungen der Formel XVII sind bekannt oder können auf an sich bekannte Weise durch Nitrierung von bekannten Phenolen oder Thiophenolen erhalten werden.

Verbindungen der Formel III sind bekannt oder können nach an sich bekannten Methoden oder analog zu an sich bekannten Methoden hergestellt werden.

Säuren der allgemeinen Formel IV

(siehe Formel IV)

worin $R^{1''''}$, $R^2$, $R^3$, $R^4$, $R^5$, $R^6$, Y und n obige Bedeutung besitzen, und deren Säurederivate sind neue Verbindungen, welche wertvolle Zwischenprodukte zur Herstellung von pharmakologisch aktiven Verbindungen, beispielsweise den Verbindungen der Formel I darstellen. Die Säuren der Formel IV können auf an sich bekannte Weise durch Hydrolyse entsprechender Ester der allgemeinen Formel XX

(siehe Formel XX)

worin $R^{1''''}$, $R^2$, $R^3$, $R^4$, $R^5$, $R^6$, Y, n und $R^9$ obige Bedeutung besitzen, erhalten werden. Sofern $R^{1''''}$ für eine $COOR^8$-Gruppe steht, müssen die Reaktionsbedingungen für die Hydrolyse der Estergruppe $COOR^9$ in der Seitenkette so schonend gewählt werden, daß eine gleichzeitige Hydrolyse der ringgebundenen $COOR^8$-Gruppe vermieden wird.

Verbindungen der Formel XX können erhalten werden, indem man Verbindungen der allgemeinen Formel XXIa

(siehe Formel XXIa)

worin $R^2$, $R^3$, Y, n und $R^9$ obige Bedeutung besitzen, mit Verbindungen der Formel VII umsetzt. Die Umsetzung kann auf an sich bekannte Weise, beispielsweise unter den in Verfahrensvariante e) zur Umsetzung von Verbindungen der Formel VI mit Verbindungen der Formel VIIa angegebenen Reaktionsbedingungen durchgeführt werden. Verbindungen der Formel XX, worin $R^{1''''}$ niederesAlkyl bedeutet, können auch wie vorstehend beschrieben aus entsprechenden Nitrilen der Formel X erhalten werden.

Verbindungen der Formel V sind neue Verbindungen, welche wertvolle Zwischenprodukte zur Herstellung von pharmakologisch aktiven Verbindungen, beispielsweise den Verbindungen der Formel I, darstellen. Sie können nach an sich bekannten Methoden hergestellt werden.

Verbindungen der allgemeinen Formel Va

(siehe Formel Va)

worin $R^2$, $R^3$, $R^4$, $R^5$, $R^6$, Y, n, Z', Q, $R^7$ und $R^8$ obige Bedeutung besitzen, können erhalten werden, indem man Verbindungen der allgemeinen Formel IIb

(siehe Formel IIb)

worin $R^2$, $R^3$, $R^4$, $R^5$, $R^6$, Y, n, Z', $R^8$ und X obige Bedeutung besitzen, mit Verbindungen der Formel III umsetzt. Die Umsetzung kann nach an sich zur Aminoalkylierung üblichen Methoden, beispieslweise bei den unter Verfahrenvariante a) zur Umsetzung von Verbindungen der Formel IIa mit Verbindungen der Formel III angegebenen Bedingungen, durchgeführt werden.

Verbindungen der allgemeinen Formel Vb

(siehe Formel Vb)

worin $R^2$, $R^3$, $R^4$, $R^5$, $R^6$, Y, n, Q, $R^7$ und $R^8$ obige Bedeutung besitzen, können erhalten werden, indem man Säuren der allgemeinen Formel IVb

(siehe Formel IVb)

worin $R^2$, $R^3$, $R^4$, $R^5$, $R^6$, Y, n und $R^8$ obige Bedeutung besitzen, mit Verbindungen der Formel III umsetzt. Die Umsetzung kann nach zur Amidbildung üblichen Methoden, beispielsweise unter den unter Verfahrensvariante b) zur Umsetzung von Verbindungen der Formel IVa mit Verbindungen der Formel III angegebenen Bedingungen, erfolgen.

Verbindungen der Formel Va, worin Z' eine Methylengruppe darstellt, können auch durch Reduktion entsprechender Verbindunge der Formel Vb mit Diboran oder einem Borhydrid/Tetrahydrofuran-Komplex in Tetrahydrofuran erhalten werden. Unter diesen Reaktionsbedingungen wird die $COOR^8$-Gruppe nicht angegriffen.

Verbindungen der allgemeinen Formel Vc

(siehe Formel Vc)

worin $R^2$, $R^3$, $R^4$, $R^5$, $R^6$, Y, n, Z, $R^{7'}$ und $R^8$ obige Bedeutung besitzen, können auch erhalten werden, indem man Verbindungen der Formel VI mit Verbindungen der allgemeinen Formel VIIb

(siehe Formel VIIb)

worin $R^4$, $R^5$, $R^6$ und $R^8$ obige Bedeutung besitzen, umsetzt. Die Umsetzung kann unter den für die Umsetzung von Verbindungen der Formel VI mit Verbindungen der Formel VIIa gemäß Verfahrensvariante e) angegebenen Bedingungen erfolgen.

Verbindungen der Formel VI sind neue Verbindungen, welche wertvolle Zwischenprodukte zur Herstellung von pharmakologisch aktiven Verbindungen darstellen. Sie können nach an sich bekannten Methoden hergestellt werden. Beispielsweise können Verbindungen der Formel VI ausgehend von Verbindungen der allgemeinen Formel XXII

(siehe Formel XXII)

worin $R^2$, $R^3$, Y, n, Z und $R^{7'}$ obige Bedeutung besitzen, erhalten werden. Hierzu werden die Verbindungen der Formel XXII durch Behandeln mit Natriumnitrit auf an sich bekannte Weise in die entsprechenden N-Nitroso-Verbindungen überführt und diese anschließend zu den Hydrazinverbindungen der Formel VI reduziert. Die Bildung der Nitrosoverbindungen und deren anschließende Reduktion können auf an sich bekannte Weise unter den vorstehend für die Herstellung von Verbindungen der Formel IX ausgehend von Verbindungen der Formel XI und die Herstellung von Verbindungen der Formel XXI ausgehend von Verbindungen der Formel XIII angegebenen Bedingungen erfolgen.

Verbindungen der allgemeinen Formel XXIIa

(siehe Formel XXIIa)

worin $R^2$, $R^3$, Y, n und $R^{7'}$ obige Bedeutung besitzen, können durch Reduktion von Verbindungen der allgemeinen Formel XXIII

(siehe Formel XXIII)

worin $R^2$, $R^3$, Y, n und $R^{7'}$ obige Bedeutung besitzen und Z'' die $CH_2$-Gruppe oder die CO-Gruppe bedeutet, erhalten werden. Die Reduktion kann auf an sich bekannte Weise unter den vorstehend für die Reduktion von Verbindungen der Formel XIV zu Verbindungen der Formel XIIIa angegebenen Bedingungen mit Diboran oder einem Borhydrid/Tetrahydrofuran-Komplex in Tetrahydrofuran erfolgen. Bei der Reduktion wird eine allfällige CO-Gruppe Z'' ebenfalls mitreduziert.

Verbindungen der Formel XXIIb

(siehe Formel XXIIb)

worin $R^2$, $R^3$, Y, n, Z' und $R^{7'}$ obige Bedeutung besitzen, können erhalten werden, indem man Verbindungen der allgemeinen Formel XXIV

(siehe Formel XXIV)

worin $R^2$, $R^3$, Y, n und Z' obige Bedeutung besitzen und X' für Halogen, vorzugsweise Brom, steht mit Verbindungen der allgemeinen Formel IIIa

(siehe Formel IIIa)

worin $R^{7'}$ obige Bedeutung besitzt, umsetzt. Die Umsetzung kann nach an sich zur Aminoalkylierung üblichen Methoden erfolgen und beispielsweise unter den für die Umsetzung von Verbindungen der Formel IIa mit Verbindungen der Formel III gemäß Verfahrensvariante a) angegebenen Reaktionsbedingungen

durchgeführt werden.

Verbindungen der Formel XXII, worin Z die CO-Gruppe bedeutet, können erhalten werden, indem man Ester der Formel XIIIa zu entsprechenden Säuren hydrolysiert und diese mit Verbindungen der Formel IIIa umsetzt.

Verbindungen der Formel XXIII, worin Z'' eine $CH_2$-Gruppe bedeutet, können erhalten werden, indem man Verbindungen der allgemeinen Formel XXV

(siehe Formel XXV)

worin $R^2$, $R^3$, Y, n und X' obige Bedeutung besitzen mit Verbindungen der Formel IIIa unter zur Aminoalkylierung üblichen Bedingungen umsetzt.

Verbindungen der Formel XXIII, worin Z'' eine CO-Gruppe bedeutet, können erhalten werden, indem man durch Hydrolyse der Ester der Formel XIV erhaltene Säuren mit Verbindungen der Formel IIIa unter zur Aminoacylierung üblichen Bedingungen, beispielsweise bei den unter Verfahrenvariante b) beschriebenen Bedingungen umsetzt.

Verbindungen der Formel XXV können erhalten werden, indem man Aminophenole bzw. Aminothiophenole der Formel XV mit Verbindungen der allgemeinen Formel XXVI

(siehe Formel XXVI)

worin $R^9$, n und X' obige Bedeutung besitzen, umsetzt. Die Umsetzung kann auf an sich bekannte Weise in einem unter den Reaktionsbedingungen inerten organischen Lösungsmittel, beispielsweise Aceton, durchgeführt werden. Zweckmäßigerweise wird eine zum Abfangen der freiwerdenden Säure geeignete anorganische Base, beispielsweise ein Alkalimetallcarbonat, zugesetzt und die Reaktion bei erhöhter Temperatur, beispielsweise Siedetemperatur des Reaktionsgemisches, durchgeführt.

Verbindungen der Formel XXIV können erhalten werden, indem man Verbindungen der Formel XIII zu den entsprechenden Alkoholen der allgemeinen Formel XXVII

(siehe Formel XXVII)

worin $R^2$, $R^3$, n und Z' obige Bedeutung besitzen, reduziert und in diesen die Hydroxygruppe in an sich bekannter Weise in ein Halogen X' überführt. Die Reduktion der Verbindungen der Formel XIII zu den Alkoholen der Formel XXVII kann beispielsweise unter den für die Herstellung der Alkohole der Formel VIIIb aus Verbindungen der Formel XXa angegebenen Reaktionsbedingungen durchgeführt werden. Die Einführung eines Halogenrestes X' kann unter den zur Herstellung von Verbindungen der Formel II aus Alkoholen der Formel VIII angegebenen Reaktionsbedingungen durchgeführt werden.

Verbindungen der Formel VII sind bekannt oder können nach an sich bekannten Methoden oder analog zu an sich bekannten Methoden hergestellt werden, beispielsweise durch Umsetzung von Verbindungen der allgemeinen Formel XXIX

(siehe Formel XXIX)

worin $R^4$, $R^5$ und $R^6$ obige Bedeutung besitzen, mit Säurechloriden der allgemeinen Formel XXVIII

(siehe Formel XXVIII)

worin $R^{1'''}$ obige Bedeutung besitzt.

Sofern $R^6$ in den Verbindungen der Formel XXIX Wasserstoff bedeutet, werden sich bei der Umsetzung mit den Säurechloriden der Formel XXVIII zunächst die entsprechenden Phenolester bilden, welche anschließend auf an sich bekannte Weise unter den Bedingungen einer Fries-Umlagerung, z. B. durch Behandeln mit Aluminiumtrichlorid in Nitromethan, in die Verbindungen der Formel VII überführt werden können.

Die Verbindungen der Formel I und ihre pharmakologisch akzeptablen Säureadditionssalze zeichnen sich durch interessante pharmakologische Eigenschaften aus und besitzen antiinflammatorische und antiallergische Wirkungen. Insbesondere zeigen die Verbindungen ein zur Behandlung von asthmatischen Beschwerden günstiges Wirkungsprofil mit geringer Toxizität und guter Verträglichkeit.

Asthma ist ein chronisches inflammatorisches Lungenleiden, welches durch episodisch auftretende, reversible Behinderungen der Atemwege charakterisiert ist. Es wird allgemein angenommen, daß die Auslösung von asthmatischen Beschwerden und Anfällen von einem als Mastzelle bekannten parenchymalen und interstitialen Zelltyp ausgeht. Diese Mastzellen enthalten vorgebildete Entzündungsmediatoren und Spasmogene, insbesondere Histamin. Sie sind auch befähigt eine Reihe von von Membranlipiden abstammenden Mediatoren neu zu synthetisieren. Mastzellen wirken auch im Verein mit einer Vielzahl von Begleitzellen, welche alle fähig sind inflammatorische und proinflammatorische Mediatoren zu synthetisieren.

Solange keine allergieauslösenden Bedingungen vorliegen, befinden sich die Mastzellen in quasi unbeteiligter Wartestellung. Der Schlüssel zu allergischen Reaktionen liegt in der Bindung von Antigenen an die membrangebundenen IgE-Antikörper auf Mastzellen. Diese Bindung vernetzt mehrere IgE-Antikörper, welches zu einer Aktivierung der Mastzelle führt. Die Mastzelle setzt daraufhin vorgeformte Mediatoren wie

Histamin frei und synthetisiert neue Mediatoren wie Leukotriene.

Da Asthma ein inflammatorisches obstruktives Lungenleiden ist, stützt sich die Therapie im wesentlichen auf zwei Wege: Erleichterung der symptomatischen Beschwerden durch Verabreichung von Bronchodilatoren wie β-Sympathicomimetica, Xanthin-Derivate und Anticholinergica; Verabreichung von antiinflammatorischen Wirkstoffen wie Dinatriumcromoglicinat und Steroiden; und gegen spezifische Mediatoren wie z. B. Histamin zielgerichtete Therapien. Eine Behandlung zur Linderung der symptomatischen Beschwerden ist bei etwa 50 % der Asthmatiker angemessen, trägt jedoch nicht dazu bei, die Ursachen, das ist die Entzündung, zu lindern. Antiinflammatorische Wirkstoffe können die Entzündung eindämmen, besitzen jedoch oft unerwünschte Nebenwirkungen und werden oft gleichzeitig mit Bronchodilatoren verabreicht. Gegen einen spezifischen Mediator zielgerichtete Therapien sind allein völlig unzureichend, da es eine Vielzahl von Mediatoren gibt.

Die erfindungsgemäßen Substanzen zeichnen sich dadurch aus, daß sie antiinflammatorisch wirksam sind und zielgerichtet gegen einen oder mehrere der drei Mediatortypen Histamin, Leukotriene und Blutplättchenaggregationsfaktor, welche nicht nur bei akuten Bronchospasmen, sondern auch bei der Aufrechterhaltung der chronischen Entzündung beteiligt sind, wirken oder auch über mediatorenspezifische Rezeptoren gegen die betreffenden Targetzellen wirksam sind. Dabei besitzen die Substanzen ein günstiges Wirkprofil, welches durch eine ausgeprägte, gegen Leukotriene gerichtete Wirkkomponente gekennzeichnet ist.

Die antiinflammatorischen und antiallergischen Eigenschaften der Verbindungen lassen sich in pharmakologischen Standardtestmethoden in vitro und in vivo nachweisen.

Beschreibung der Testmethoden

1. Bestimmung der Hemmung der durch Histamin induzierten anaphylaktoiden cutanen Reaktion.

Die Bestimmung der Hemmung der durch Histamin induzierten anaphylaktoiden cutanen Reaktion wird an Sprague-Dawley-Ratten mit einem Körpergewicht von 150-180 g durchgeführt.

Zur Bestimmung der histamininduzierten anaphylaktoiden Hautreaktion werden den Tieren an einer Flanke 50 µl einer 0,8 mg/ml Histamin enthaltenden physiologischen Natriumchlorid-lösung intradermal injiziert. Direkt danach wird den Tieren eine Lösung von 26,4 mg/kg eines blauen Farbstoffes (Evan's Blue) i.v. appliziert.

Die Testsubstanzen werden in destilliertem Wasser, welches 1 Vol.-% Dimethylformamid und 1 Vol.-% Tween$^R$20 (= Polyoxyäthylen(20)sorbitanmonolaurat) enthält, gelöst. Eine Stunde vor der Histamininjektion erhält jedes Tier 2 x $10^{-5}$ Mol/kg Testsubstanz jeweils in 0,5 ml Lösung oral appliziert. Eine Kontroll-gruppe erhält zum Vergleich nur das Lösungsmittel.

Die histamininduzierten, anaphylactoiden Reaktionen, welche sich durch Ödembildung, Schwellung und Exsudation von blauem Farbstoff zeigen, werden 30 Minuten nach der Reizung durch die Histamin-Injektion ausgewertet. Dies geschieht durch visuelle Bestimmung des Ausmaßes des Austritts von blauem Farbstoff an den Stellen der Ödembildung. Mit Hilfe von Vergleichsskalen wird die durch die Testsubstanzen hervorgerufene prozentuale Hemmung der anaphylactoiden Reaktionen im Vergleich zu den Reaktionen der nicht mit Testsubstanz behandelten Kontrolltiere bestimmt.

Die nach der vorstehenden Testmethode mit Verbindungen der Formel I erhaltenen Ergebnisse werden in der nachfolgenden Tabelle A wiedergegeben. Die für die Verbindungen der Formel I angegebenen Beispielsnummern beziehen sich auf die nachfolgenden Herstellungsbeispiele.

Tabelle A

| Test-subst. Bsp: Nr. | Hemmwirkung gegenüber cutanen histamin-induzierten anaphylactoiden Reaktionen in der Ratte % Hemmung bei Dosis von 2 x $10^{-5}$ Mol/kg p.o. |
|---|---|
| 15 | 32 |
| 19 | 45 |
| 25 | 30 |
| 27 | 55 |
| 29 | 46 |
| 30 | 47 |
| 31 | 58 |
| 32 | 57 |
| 33 | 39 |
| 39 | 32 |
| 41 | 60 |
| 42 | 36 |
| 43 | 32 |
| 44 | 37 |

2. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder stark toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis in der nachstehenden Tabelle B angegeben.

Tabelle B

| Testsubstanz Beispiel Nr. | Minimale toxische Dosis mg/kg Maus p.o. |
|---|---|
| 19 | > 300 |
| 5 | > 300 |

3. Bestimmung der Anti-P.A.F.-Wirkung in vitro.

P.A.F. (= Platelet Activating Factor = Blutplättchenaggregationsfaktor) ist ein phospholipidischer Mediator, welcher eine Vielzahl von Wirkungen besitzt. Die Aktivierung der Blutplättchenaggregation führt zur Induzierung langanhaltender Bronchokontraktionen und Überreaktivität der Luftwege.

In diesem Test wird nach der von Mikashima et al. beschriebenen Methode (Jap. J. Pharmacol. 44 - (1987) 387-391) die Wirkung der Testsubstanzen auf eine durch Zugabe von P.A.F. induzierte Blutplättchenaggregation in einer aus Kaninchenblut gewonnenen Blutplättchensuspension untersucht.

Es wird eine Suspension von aus Kaninchenblut stammenden Blutplättchen verwendet, welche 4 x $10^9$ Blutplättchen/ml in einer auf pH 7,4 eingestellten, modifizierten Tyrode-Pufferlösung (= Tyrode-Lösung*mit Zusatz von 1,3 mM/l $CaCl_2$ und 2,5 g/l Gelatine) enthält. Die Blutplättchen werden aus 10 ml Blutproben von jeweils drei Kaninchen (Neuseelandhybriden, Körpergewicht 3 - 4 kg) angereichert. Hierzu werden die Blutproben mit Ethylendiamintetraessigsäure behandelt und nach der Methode von Artley et al. (Brit. J. Haematol. 19 (1970), 7-17) gewaschen. Sodann wird durch Zentrifugieren (20 Minuten bei 400 x g) zunächst

* Tyrodelösung = wäßrige Lösung enthaltend pro Liter 136,9 mMol NaCl, 2,68 mMol KCl, 2,31 mMol $CaCl_2$, 1,0 mMol $MgCl_2$, 11,9 mMol $NaHCO_3$, 1,45 mMol $NaH_2PO_4$ und 5,55 mMol Glucose.

ein blutplättchenreiches Plasma abgetrennt. Durch nochmaliges Zentrifugieren für 15 Minuten bei 1400 x g werden die Blutplättchen von dem Plasma getrennt. Nach dem Zentrifugieren werden die im Sediment verbleibenden Blutplättchen in einer Tyrode-Pufferlösung (jedoch ohne Calcium) resuspendiert. Sodann werden 0,4 mMol Lysinacetylsalicylat zugegeben, und nach 15 Minuten werden die Blutplättchen nochmals sedimentiert. Das Sediment wird in der vorgenannten modifizierten Tyrode-Pufferlösung resuspendiert, und die Anzahl der Blutplättchen in der erhaltenen Suspension wird auf den gewünschten Gehalt eingestellt.

Es wird eine 40 x $10^{-9}$-molare P.A.F.-Lösung als Reagenz eingesetzt. Diese Lösung wird aus einer 1,8 x $10^{-3}$-molaren Vorratslösung in Chloroform hergestellt. Hierzu wird eine 10 $\mu$l Probe der Vorratslösung zur Trockne eingedampft und wieder in 180 $\mu$l der modifizierten Tyrode-Pufferlösung, welcher 0,25 % von Lipiden befreites Rinderserum-Albumin zugesetzt worden war, gelöst. Daraus werden sodann $10^{-5}$-molare Arbeitslösungen hergestellt und gefroren aufbewahrt. Für Tests werden Proben dieser Lösungen entsprechend verdünnt.

Zur Testdurchführung werden in einer mit einem kleinen Magnetrührer versehenen Aggregationsröhrchen zu 330 $\mu$l der modifizierten Tyrode-Pufferlösung unter Rühren (1000 UpM) 50 $\mu$l der Blutplättchensuspension und 10 $\mu$l einer 40 x $10^{-5}$-molaren Lösung der zu untersuchenden Verbindung gegeben. Dies entspricht einer Endkonzentration an Testsubstanz von $10^{-5}$ Mol/l. Nach 90 Sekunden Vorinkubationszeit werden 10 $\mu$l der P.A.F.-Zubereitung hinzugefügt. Während 4-5 Minuten wird die in den Aggregationsröhrchen auftretende Aggregation mit Hilfe eines computerisierten Aggregometers gemessen.

Die in den nur Blutplättchensuspension enthaltenden Teströhrchen auftretende Aggregation wird als 0 % gewertet. Die in den Blutplättchensuspension und P.A.F.-Zubereitung enthaltenden Teströhrchen, in denen eine durch P.A.F. induzierte Blutplättchenaggregationsverstärkung stattfindet, auftretende Aggregation wird als 100 % gewertet. Die nach Zusatz der Testsubstanzen noch auftretende Aggregation wird gemessen und daraus die erfolgte Hemmung der durch P.A.F. induzierten Aggregationsverstärkung in % berechnet.

Die nach der vorstehenden Methode mit den Verbindungen der Formel I erhaltenen Ergebnisse werden in der nachfolgenden Tabelle C wiedergegeben.

**Tabelle C**

| Test subst. Bsp. Nr. | Anti-P.A.F.-Wirkung in vitro. % Hemmung der P.A.F.-induzierten Aggregation von Blutplättchen aus Kaninchenblut bei einer Testsubstanzkonzentration von $10^{-5}$ Mol/l |
|---|---|
| 1 | 80 |
| 2 | 97 |
| 3 | 92 |
| 4 | 99 |
| 5 | 100 |
| 6 | 100 |
| 9 | 67 |
| 12 | 93 |
| 13 | 94 |
| 14 | 100 |
| 15 | 100 |
| 17 | 99 |
| 18 | 78 |
| 19 | 71 |
| 20 | 92 |

| | |
|---|---|
| 22 | 84 |
| 23 | 66 |
| 25 | 91 |
| 26 | 82 |
| 28 | 83 |
| 29 | 50 |
| 30 | 69 |
| 31 | 52 |
| 32 | 79 |
| 33 | 43 |
| 34 | 77 |
| 37 | 48 |
| 41 | 99 |
| 42 | 40 |
| 43 | 95 |
| 44 | 99 |
| 48 | 69 |
| 51 | 65 |
| 54 | 65 |
| 63 | 80 |
| 65 | 52 |
| 67 | 97 |
| 68 | 96 |
| 69 | 97 |
| 70 | 93 |
| 71 | 84 |
| 72 | 79 |

4. In vitro-Bestimmung der 5-Lipoxygenase-Hemmung.

4a. In vitro-Bestimmung der Hemmung der 5-Lipoxygenase-Aktivitätin polymorphonuklearen Leukocyten der Ratte.

In Zellmembranen enthaltene Arachidonsäure wird nach Aktivierung der polymorphonuklearen Leukocyten (= PMN) freigesetzt. Dabei werden unter Einwirkung des Enzyms 5-Lipoxygenase (=5-LO) Arachidonsäuremetaboliten synthetisiert, die durch $LTA_4$-Hydrolase zu Leukotrien $B_4$ (= $LTB_4$) umgewandelt werden können. $LTB_4$ ist ein Hauptmetabolit des bei Aktivierung des Enzyms 5-LO durch den inflammatorischen Reiz in der Zellmembran ablaufenden Arachidonsäurestoffwechsels. In in vitro-Systemen wird dieser Metabolit aus PMNs sekretiert.

Zur Untersuchung der 5-lipoxygenasehemmenden Eigenschaften wird die Hemmwirkung der Testsubstanzen auf die Biosynthese des Arachidonsäurederivates Leukotrien $B_4$ (= $LTB_4$) an peritonealen PMNs der Ratte in vitro bestimmt. Hierzu wird der Gehalt an $LTB_4$ in einem Kulturmedium von peritonealen PMNs

der Ratte nach Stimulation durch Calcimycin (= Calcium-Ionophor A23187) wie von J. Gillard et al., Can. J. Physiol. Pharmacol. 67 (1989), 17 beschrieben bestimmt.

Eine peritoneale PMNs von männlichen Sprague-Dawley Ratten (Körpergewicht 200 bis 250 g) enthaltende Zellsuspension wird auf bekannte Weise wie folgt gewonnen: Den Tieren werden 6 ml einer 27,9 g/l Natriumthioglycollat enthaltenden Lösung i.p. injeziert, um durch diesen inflammatorischen Reiz die PMN-Einwanderung anzuregen. Nach 22 Stunden werden die Tiere durch lethale Ethernarkose getötet. Sodann werden in die Bauchhöhle 20 ml einer Salzlösung nach Hanks (= Hank's Balanced Salt Solution = HBSS) jedoch ohne $Ca^{++}$- und $Mg^{++}$-ionen und mit 0,2 Gew.-% Natriumcitrat injiziert. Die Suspension der akkumulierten Zellen wird aus der Bauchhöhle entnommen und die Zellen werden durch Zentrifugieren (10 Minuten bei 400 x g) abgetrennt. Durch nochmaliges Zentrifugieren in Gegenwart von Ficoll-Paque$^R$,(= Metrizoinsäurezubereitung, d = 1077, Hersteller Fa. Pharmacia) werden die Zellen gereinigt, sodann zweimal mit $Ca^{++}$- und $Mg^{++}$-ionen enthaltender HBSS gewaschen und in $Ca^{++}$- und $Mg^{++}$-ionen enthaltender, mit 10 mM Hepes (= Hydroxyethylpiperazinoethansulfonsäure) gepufferter HBSS resuspendiert.

Zur Testdurchführung werden Proben von jeweils 0,7 ml der PMN-Suspension (= 5 x $10^6$ Zellen) in Plastikröhrchen mit 10 µM der Testsubstanz in Dimethylformamid oder als Kontrollversuch nur mit Dimethylformamid 15 Minuten lang bei 22 °C inkubiert. Die $LTB_4$-Synthese wird in den Proben durch Zugabe von Calcimycin (End-Konzentration 5 µM) initiiert und 10 Minuten lang bei 22 °C inkubiert. Kontrollblindproben wird nur Lösungsmittel zugesetzt. Nach 10 Minuten wird die Reaktion durch Zugabe von 0,3 ml kaltem Methanol beendet. Die Proben werden 1 Stunde bei 4 °C gehalten und dann zentrifugiert (5 Minuten bei 10 000 x g). Die überstehende Flüssigkeit wird mit Hilfe von Hochdruck-Flüssigkeitschromatographie mit umgekehrten Phasen (= Reverse Phase HPLC) auf ihren Gehalt an $LTB_4$ untersucht. Hierzu werden als Adsorbens eine 5µM-C18-silanisierte Kieselgelsäule (Hichrom$^R$ $ODS_2$, 250 x 4,6 mm) und als Eluens ein mit Ammoniak auf pH 5,7 eingestelltes Gemisch Methanol/Wasser/Essigsäure 72:28:0,02 v/v/v mit einer Durchlaufgeschwindigkeit von 1 ml/min eingesetzt. Das $LTB_4$ wird durch Vergleich mit zur gleichen Zeit eluierenden authentischen Standardlösungen identifiziert und bei einer Wellenlänge von 270 nm bestimmt. Die $LTB_4$-Gehalte werden aus dem Vergleich mit kalibrierten Standardlösungen bestimmt, und daraus die Hemmwirkung der Testsubstanzen in % Hemmung der $LTB_4$-Synthese im Vergleich zu den keine Testsubstanz enthaltenden Kontrollproben berechnet. Die mit dieser Testmethode erhaltenen Ergebnisse werden in der nachfolgenden Tabelle D wiedergegeben.

**Tabelle D**

| Test Subst. Bsp. Nr. | In vitro %-Hemmwirkung auf die $LTB_4$-Bildung in durch Calcium-ionophor A23187 stimulierten Ratten-PMNs bei einer Testsubstanzkonzentration von $10^{-5}$ Mol/l |
|---|---|
| 2 | 89 |
| 3 | 96 |
| 4 | 100 |
| 9 | 86 |
| 12 | 100 |
| 13 | 98 |

| | |
|---|---|
| 14 | 90 |
| 15 | 94 |
| 16 | 60 |
| 19 | 45 |
| 20 | 75 |
| 21 | 42 |
| 23 | 92 |
| 24 | 73 |
| 26 | 67 |
| 27 | 90 |
| 28 | 48 |
| 30 | 100 |
| 31 | 81 |
| 32 | 98 |
| 33 | 90 |
| 34 | 95 |
| 35 | 79 |
| 36 | 89 |
| 37 | 100 |
| 38 | 97 |
| 39 | 98 |
| 40 | 82 |
| 41 | 100 |
| 43 | 66 |
| 44 | 100 |
| 49 | 88 |
| 52 | 53 |
| 55 | 63 |
| 56 | 76 |
| 57 | 85 |
| 58 | 64 |
| 62 | 66 |
| 65 | 64 |
| 67 | 100 |
| 68 | 90 |
| 69 | 100 |

| | |
|---|---|
| 70 | 68 |
| 71 | 100 |
| 72 | 100 |
| 73 | 100 |
| 74 | 100 |
| 75 | 51 |

4b. In vitro-Bestimmung der Hemmwirkung auf die aus peritonealen PMNs des Meerschweinchens angereicherte 5-Lipoxygenase.

Eine 5-Lipoxygenasehemmung kann durch direkte Kemmwirkung der Substanzen auf das Enzym und/oder durch eine Störung der Aktivierung des Enzyms verursacht werden. Während in dem Test 4a die 5-Lipoxygenase-Hemmung der Substanzen in PMN-Zellen der Ratte untersucht wird, wird im Test 4b die Hemmwirkung der Substanzen auf freie, aus PMN-Zellen des Meerschweinchens angereicherte 5-LO nach der Methode von D. Aharony et al., J. Biol. Chem. 261 (1986), 11512-11519 (1986) untersucht.

Die 5-LO wird auf an sich bekannte Weise aus PMNs von männlichen Dunkin-Hartley Meerschweinchen (Körpergewicht 400 bis 500 g) wie folgt angereichert. Den Tieren werden 10 ml einer 27,9 g/l Natriumthioglycollat enthaltenden Lösung i.p. injiziert, um durch diesen inflammatorischen Reiz die PMN-Einwanderung anzuregen. Nach 22 Stunden werden die Tiere durch lethale Ethernarkose getötet. Sodann werden in die Bauchhöhle 70 ml HBSS ohne $Ca^{++}$- und $Mg^{++}$-ionen und mit 0,2 Gew.-% Natriumcitrat injiziert. Die Zellsuspension (> 80 % der Zellen sind PMNs) wird aus der Bauchhöhle entnommen und durch ein Käsereifiltertuch filtriert und zweimal mit einer 50 mM Kaliumphosphatpufferlösung von pH 7,4, welche 0,5 mM Ethylendiamintetraessigsäure, 1 Gew.-% Gelatine und 20 µM Indomethacin enthält, gewaschen. Die Zellen werden resuspendiert und die $10^8$ Zellen/ml enthaltende PMN-suspension wird im Eisbad dreimal je 15 Sekunden homogenisiert in einem auf hohe Geschwindigkeit eingestellten Homogenisator vom Typ Labsonic$^R$. Die erhaltene Suspension wird bei 4 °c zentrifugiert (15 Minuten bei 10 000 x g). Die überstehende Flüssigkeit wird als Lösung des freien 5-LO-Enzyms für die Untersuchung eingesetzt.

Die Versuche werden mit 0,625 ml Proben dieser Enzymlösung bei 37 °C über 7 Minuten durchgeführt. Für die Versuche werden die Enzymlösung (= Äquivalent von 2,5 x $10^7$ Zellen), 0,5 mM Glutathion, 1 mM ATP und 0,3 mM Calciumchlorid in Reagenzgläser gegeben. Zu den Proben werden 10 µMol der Testsubstanz in soviel Dimethylformamid gelöst gegeben, daß die Dimethylformamid-Konzentration in jedem Reagenzglas 0,5 % beträgt. Den Kontrollproben wird nur Dimethylformamid zugefügt. Nach 2 Minuten wird die Bildung von 5-(S)-Hydroxy-6,8,11,14-eicosatetraensäure (= 5-HETE) durch Zugabe von 10 µM Arachidonsäure eingeleitet. Nach 5 Minuten wird die Reaktion durch Zugabe von 0,625 ml eines Gemisches Methylacetat/Methanol/0,2 M Zitronensäure 15:2:0,5 v/v/v beendet. 5-HETE wird zweimal unter Verwendung dieses Lösungsmittelgemisches extrahiert. Die 5-HETE-haltigen Phasen werden vereinigt und unter Stickstoffatmosphäre zur Trockne eingedampft. Der Rückstand wird in einem Gemisch Methanol/Wasser/Essigsäure 75:21:0,02 v/v/v mit pH 5,7 aufgenommen und die Lösung wird mit Hilfe von Reverse phase HPLC auf ihren Gehalt an 5-HETE untersucht. Hierzu werden als Adsorbens eine 5 µM-C18-silanisierte Kieselgelsäule (Hichrom$^R$ $ODS_2$, 250 x 4,6 mm) und als Eluens ein mit Ammoniak auf pH 5,7 eingestelltes Gemisch Methanol/Wasser/Essigsäure 75:21:0,02 mit einer Durchlaufgeschwindigkeit von 1 ml/min eingesetzt. 5-HETE, welches nach 20 Minuten als einziger Peak eluiert wird, wird durch Vergleich mit zur gleichen Zeit eluierten Standardlösungen identifiziert und unter Verwendung eines UV-Lichtdetectors bei einer Wellenlänge von 235 nm bestimmt. Die quantitative Bestimmung erfolgt durch Vergleich der erhaltenen Peak-Fläche mit bei den kalibrierten Standardlösungen auftretenden Peak-Flächen. Aus den bestimmten 5-HETE-Gehalten der Proben wird die Hemmwirkung der Testsubstanzen in % Hemmung der 5-HETE-Synthese im Vergleich zu den keine Testsubstanz enthaltenden Kontrollproben berechnet. Die mit dieser Versuchsmethode erhaltenen Ergebnisse werden in der nachfolgenden Tabelle E wiedergegeben.

**Tabelle E**

| Test. subst. Bsp. Nr. | In vitro % Hemmwirkung auf durch freie 5-LO bewirkte 5-HETE-Bildung bei einer Testsubstanzkonzentration von $10^{-5}$ Mol/l |
|---|---|
| 3 | 53 |
| 4 | 100 |
| 9 | 88 |
| 12 | 100 |
| 11 | 95 |
| 13 | 99 |
| 15 | 94 |
| 16 | 57 |
| 17 | 100 |
| 18 | 100 |
| 19 | 46 |
| 20 | 100 |
| 21 | 30 |
| 22 | 97 |
| 23 | 93 |
| 25 | 92 |
| 26 | 96 |
| 27 | 50 |
| 28 | 44 |
| 29 | 67 |
| 34 | 97 |
| 35 | 55 |
| 36 | 65 |
| 38 | 100 |
| 39 | 100 |
| 40 | 56 |
| 41 | 98 |
| 42 | 55 |
| 43 | 77 |
| 44 | 98 |
| 49 | 99 |
| 54 | 63 |
| 59 | 70 |

| | |
|---|---|
| 61 | 74 |
| 62 | 77 |
| 65 | 99 |
| 67 | 100 |
| 69 | 100 |
| 71 | 100 |
| 72 | 100 |
| 73 | 100 |
| 74 | 100 |
| 75 | 100 |

4c. In vitro-Bestimmung der 5-Lipoxygense-Hemmung in menschlichem Blut.

Es wird die Hemmwirkung der Testsubstanzen auf die nach Stimulation durch Calcimycin stattfindende 5-LO-Aktivität in menschlichem Gesamtblut nach der von P. Gresele et al. (Biochem. Biophys. Commun. 137 (1986), 334) beschriebenen Methode untersucht.

Zur Testdurchführung wird menschliches Blut in sterilen 50 ml Teströhrchen, welche 10 Einheiten Heparin/ml Blut enthalten, gesammelt und 5-10 Minuten bei 37 °C inkubiert. Zu 2 ml Proben dieses Blutes werden jeweils solche Mengen an Testsubstanz, gelöst in 10 µl Dimethylformamid, gegeben, daß in den Proben eine Endkonzentration an Testsubstanz von 10 µMol/l erreicht wird. Zu Kontrollproben werden nur 10 µl des Lösungsmittels gegeben. Die Proben werden dann 10 Minuten bei 37 °C inkubiert. Zur Stimulierung der $LTB_4$-Synthese werden den Proben dann eine solche Menge an Calcimycin, gelöst in 200 µl Plasma, zugegeben, daß eine Calcimycin-Konzentration von 60 µMol/l erreicht wird, und die Proben werden weitere 30 Minuten bei 37 °C inkubiert. Dann wird die Reaktion durch Zugabe von 200 µl einer Ethylenglycoltetraacetatlösung (entsprechend einer Ethylenglycolacetat-Konzentration in der Probe von 100 mMol/l) beendet. Die Proben werden 10 Minuten in einem Eisbad gehalten und dann zentrifugiert (5 Minuten bei 1000 x g). Die überstehenden Flüssigkeiten, welche das während der Reaktionszeit gebildete $LTB_4$ enthalten, werden jeweils abgetrennt und zur Fällung der darin enthaltenen Proteine mit 3 ml Aceton versetzt und 45 Minuten bei 4 °C gehalten. Dann wird nochmals zentrifugiert (5 Minuten bei 1500 x g), die überstehenden Flüssigkeiten abgetrennt, und darin der Gehalt an $LTB_4$ mittels eines enzymatischen Immunassay (= EIA) auf an sich bekannte Weise bestimmt. Aus den gemessenen $LTB_4$-Werten wird die Hemmwirkung der Testsubstanzen in menschlichem Gesamtblut in % Hemmung der $LTB_4$-Synthese im Vergleich zu den keine Testsubstanz enthaltenden Kontrollproben berechnet. Die mit dieser Testmethode erhaltenen Ergebnisse werden in der nachfolgenden Tabelle F wiedergegeben.

Tabelle F

| Test. subst. Bsp. Nr. | In vitro % Hemmwirkung auf die $LTB_4$-Bildung in durch Calcimycin stimuliertem menschlichem Blut bei einer Testsubstanzkonzentration von $10^{-5}$ Mol/l |
|---|---|
| 4 | 86 |
| 41 | 100 |
| 67 | 100 |
| 69 | 95 |
| 71 | 100 |
| 72 | 95 |
| 73 | 100 |
| 74 | 100 |
| 75 | 96 |

Aufgrund ihrer vorstehend beschriebenen Wirkungen sind die Verbindungen der Formel I als antiinflammatorisch und antiallergisch wirksame Arzneimittel für größere Säugetiere, insbesondere Menschen, geeignet zur Behandlung von entzündlichen und allergischen Erkrankungen. Die erfindungsgemäßen oral wirksamen Verbindungen können in mehrfacher Hinsicht wirken, da sie gegen mehrere der Hauptmediatoren, welche an entzündlichen Vorgängen und asthmatischen Beschwerden beteiligt sind, wirksam sind.

Aufgrund dieses Wirkungsprofils ist davon auszugehen, daß die erfindungsgemäßen Verbindungen im Rahmen der Behandlung von allergisch bedingten und nicht allergisch bedingten Asthmabeschwerden nicht nur die mit asthmatischen Erkrankunkungen verbundenen symptomatischen Beschwerden lindern, sondern auch die damit verbundene Entzündung mindern können. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 10 bis 250 mg pro Einzeldosis.

Als Heilmittel können die Verbindungen der Formel I mit üblichen pharmazeutischen Hilfsstoffen in galenischen Zubereitungen, wie z. B. Tabletten, Kapseln, Suppositorien oder Lösungen, enthalten sein. Diese galenischen Zubereitungen können nach an sich bekannten Methoden hergestellt werden unter Verwendung üblicher fester Trägerstoffe wie z. B. Milchzucker, Stärke oder Talkum oder flüssigen Paraffinen und unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Struktur der neuen Substanzen wurde durch spektroskopische Untersuchungen, insbesondere durch eine Analyse der IR- und NMR-Spektren, sowie durch Elementaranalyse gesichert. Die Reinheit der Zwischenprodukte wurde dünnschichtchromatographisch überwacht.

**Beispiel 1:**

2,3-Dihydro-5-(4-methoxyphenyl)-6-methyl-2-[2-(4-(4-methylpyridin-2-yl)-piperazin-1-yl)-ethyl]-pyrrolo[1,2,3-de]-1,4-benzoxazin.

A) Eine Mischung von 76,4 g 2-Aminophenol und 68,6 g Maleinsäureanhydrid in 1 l Methanol wurde bei Raumtemperatur gerührt. Nach Zugabe von 100 ml Triethylamin löste sich die gelbe Masse und die Lösung wurde 7 Stunden bei Rückflußtemperatur erhitzt. Dann wurde das Methanol unter vermindertem Druck abgedampft und das verbleibende Öl wurde in Eiswasser gegossen. Das Gemisch wurde mit Dichlormethan extrahiert, der organische Extrakt über Natriumsulfat getrocknet und eingedampft. Der verbleibende Rückstand wurde aus Ethanol umkristallisiert. Hierbei wurden 47,5 g (3-Oxo-2,3-dihydro-4H-1,4-benzoxazin-2-yl)-essigsäuremethylester als weiße Kristalle mit einem Schmelzpunkt von 140 °C erhalten.

B) 12 g Lithiumaluminiumhydrid wurden langsam unter Kühlen auf 0 °C in 300 ml Tetrahydrofuran gegeben. Dann wurde tropfenweise eine Lösung von 18 g des vorstehend erhaltenen Esters in 200 ml Tetrahydrofuran zugegeben. Das Reaktionsgemisch wurde anschließend für 2 Stunden am Rückfluß erhitzt. Zur Aufarbeitung wurden langsam 12 ml Wasser und anschließend 100 ml Tetrahydrofuran, 12 ml 4 N Natriumhydroxidlösung und nochmals 30 ml Wasser zugegeben. Die Lösung wurde filtriert und der Rückstand mit Diethylether nachgewaschen. Das Filtrat wurde eingeengt und der verbleibende Rückstand wurde in Dichlormethan gelöst. Die Lösung wurde über Natriumsulfat getrocknet, filtriert und das Lösungsmittel abgedampft. Es wurden 14,1 g 4H-2,3-Dihydro-2-(2-hydroxyethyl)-1,4-benzoxazin als Öl erhalten.

C) 12 g des vorstehend erhaltenen Alkoholes wurden in eine Mischung aus 100 ml Wasser, 50 g Eis und 10 ml 12 N Salzsäurelösung gegeben. Zu der Lösung wurde unter Kühlung auf 0 bis 5 °C tropfenweise eine Lösung von 4,3 g Natriumnitrit in 10 ml Wasser gegeben. Nach Beendigung der Zugabe stieg die Temperatur des Reaktionsgemisches auf 20 °C an. Die erhaltene Paste wurde mit Toluol extrahiert und der organische Extrakt wurde über Natriumsulfat getrocknet und eingeengt. Es wurden 14,6 g 2,3-Dihydro-2-(2-hydroxyethyl)-4-nitroso-1,4-benzoxazin als Öl erhalten.

D) 21 g der vorstehend erhaltenen Nitrosoverbindung wurden langsam in 100 ml 8 g Lithiumaluminiumhydrid enthaltendes Tetrahydrofuran gegeben. Das Reaktionsgemisch wurde eine halbe Stunde bei Raumtemperatur gerührt und dann auf -10 °C abgekühlt. Zur Aufarbeitung wurden nacheinander 8 ml Wasser, 10 ml Tetrahydrofuran und 15 ml 10%iger Natriumhydroxidlösung gegeben. Die Lösung wurde filtriert und der Rückstand mit Dichlormethan nachgewaschen. Das Filtrat wurde über Natriumsulfat getrocknet und eingeengt. Es wurden 17,3 g 4-Amino-2,3-dihydro-2-(2-hydroxyethyl)-1,4-benzoxazin als gelbes Öl erhalten.

E) 17,3 g der vorstehend erhaltenen Hydrazinverbindung und 16,1 g 4-Methoxypropiophenon wurden in 60 ml Ethanol gelöst. Das Reaktionsgemisch wurde 2,5 Stunden lang unter Stickstoffatmosphäre am Rückfluß erhitzt. Dann wurden bei Rückflußtemperatur 12 ml 12 N Salzsäurelösung tropfenweise über einen Zeitraum von einer halben Stunde verteilt zugegeben. Anschließend wurde das Reaktionsgemisch abgekühlt. Zur Aufarbeitung wurden 100 ml Wasser zugefügt und mit Dichlormethan extrahiert. Der Dichlormethanextrakt wurde über Natriumsulfat getrocknet und eingeengt. Es wurden 16 g 2,3-Dihydro-2-(2-hydroxyethyl)-5-(4-methoxyphenyl)-6-methyl-pyrrolo[1,2,3-de]-1,4-benzoxazin als Öl erhalten.

F) Zu einer auf ca. 0 °C gekühlten Lösung von 34 g des vorstehend erhaltenen Alkohols in 80 ml Pyridin wurden 25 g Tosylchlorid zugegeben. Dann wurde das Reaktionsgemisch 5 Stunden lang auf 60 °C erhitzt. Zur Aufarbeitung wurde das Reaktionsgemisch in Wasser gegossen und mit Dichlormethan extrahiert. Der Extrakt wurde über Natriumsulfat getrocknet und eingeengt. Es wurden 47,5 g 2,3-Dihydro-2-[2-(p-toluolsulfonyloxy)-ethyl]-5-(4-methoxyphenyl)-6-methyl-pyrrolo[1,2,3-de]-1,4-benzoxazin als Öl erhalten.

G) Eine Mischung aus 15,5 g der vorstehend erhaltenen Tosylverbindung und 6 g 1-(4-Methylpyridin-2-yl)-piperazin wurde in 5 ml Dimethylformamid 6 Stunden lang auf 90 °C erhitzt. Zur Aufarbeitung wurde das Reaktionsgemisch dann in 50 ml Wasser gegossen. Die Titelverbindung wurde aus dem Gemisch mit Dichlormethan extrahiert und durch Säulenchromatographie unter Verwendung von Ethylacetat/Cyclohexan 1:1 gereinigt und anschließend aus Ethanol kristallisiert. Es wurden 2,3 g der Titelverbindung als weißes Pulver mit einem Schmelzpunkt von 165 °C erhalten.

**Beispiel 2:**

2,3-Dihydro-5-(4-hydroxyphenyl)-6-methyl-2-[2-(4-(4-methylpyridin-2-yl)-piperazin-1-yl)-ethyl]-pyrrolo[1,2,3-de]-1,4-benzoxazin.

1,9 g 2,3-Dihydro-5-(4-methoxyphenyl)-6-methyl-2-[2-(4-(4-methylpyridin-2-yl)-piperazin-1-yl)-ethyl]-pyrrolo-[1,2,3-de]-1,4-benzoxazin (Herstellung siehe Beispiel 1)) wurden in 5 ml wasserfreiem Dichlormethan unter Stickstoffatmosphäre gelöst. Die Lösung wurde auf 0 bis 5 °C abgekühlt und dann wurden 1,5 g Bortribromid auf einmal zugegeben. Das Reaktionsgemisch wurde anschließend 3 Stunden bei Raumtemperatur gerührt. Dann wurde das Reaktionsgemisch wieder auf 0 bis 5 °C abgekühlt, und es wurden 10 ml Methanol zugegeben, das Reaktionsgemisch nochmals eine halbe Stunde bei Raumtemperatur gerührt und dann eingedampft. Der Rückstand wurde in 20 ml wäßriger Ammoniaklösung (pH = 8/9) gelöst und die Lösung wurde mit Dichlormethan extrahiert. Die Extrakte wurden getrocknet, filtriert und eingeengt. Die als Rückstand verbleibende rohe Titelverbindung wurde durch Säulenchromatographie unter Verwendung von Toluol/Ethanol 95:5 gereinigt und aus Isopropanol kristallisiert. Es wurden 1,5 g 2,3-Dihydro-5-(4-hydroxyphenyl)-6-methyl-2-[2-(4-(4-methylpyridin-2-yl)-piperazin-1-yl)-ethyl]-pyrrolo[1,2,3-de]-1,4-benzoxazin•0,5 $H_2O$ als weißes Pulver mit einem Schmelzpunkt von 213 bis 214 °C erhalten.

**Beispiel 3:**

2,3-Dihydro-5-(4-hydroxyphenyl)-7-methoxy-6-methyl-2-[2-(4-(4-methylpyridin-2-yl)-piperazin-1-yl)-ethyl]-pyrrolo[1,2,3-de]-1,4-benzoxazin.

A) Zu 12,4 g 4-Methoxyphenol in 120 ml Tetrahydrofuran wurden unter Rühren bei Raumtemperatur tropfenweise 6,3 g Salpetersäure gegeben. Dann wurde das Reaktionsgemisch eine halbe Stunde lang gerührt. Anschließend wurde das Lösungsmittel eingedampft und der verbleibende Rückstand chromatographisch unter Verwendung von Dichlormethan als Elutionsmittel gereinigt. Es wurden 6,2 g 2-Nitro-4-methoxyphenol als gelbes Pulver mit einem Schmelzpunkt von 72 °C erhalten.

B) 40 g 2-Nitro-4-methoxyphenol wurden unter Stickstoffatmosphäre im Dunkeln in einem Gemisch von 200 ml Wasser und 200 ml 35%iger Natriumhydroxidlösung gerührt. Zu dem Reaktionsgemisch wurden 100 g Natriumthiosulfat gegeben und das Gemisch wurde weitere 2 Stunden bei Raumtemperatur gerührt. Zur Aufarbeitung wurde das Reaktionsgemisch durch Zugabe von 12 N Salzsäurelösung neutralisiert und mit viermal 500 ml Ethylacetat extrahiert. Die organischen Extrakte wurden mit zweimal 500 ml Wasser gewaschen, getrocknet, filtriert und eingedampft. Es wurden 30 g 2-Amino-4-methoxyphenol als Rückstand erhalten und ohne weitere Reinigung weiterverarbeitet.

C) 30 g 2-Amino-4-methoxyphenol wurden mit Maleinsäureanhydrid nach der in Beispiel 1A) beschriebenen Methode umgesetzt. Es wurden 13 g (6-Methoxy-3-oxo-2,3-dihydro-4H-1,4-benzoxazin-2-yl)-essigsäuremethylester als Öl erhalten.

D) 13 g des vorstehend erhaltenen Esters wurden nach der in Beispiel 1B) beschriebenen Methode mit Lithiumaluminiumhydrid in Tetrahydrofuran reduziert. Es wurden 10,5 g 4H-2,3-Dihydro-2-(2-hydroxyethyl)-6-methoxy-1,4-benzoxazin als Öl erhalten.

E) 10,5 g des vorstehend erhaltenen Produktes wurden nach der in Beispiel 1C) beschriebenen Methode nitrosiert. Es wurden 8 g 2,3-Dihydro-2-(2-hydroxyethyl)-6-methoxy-4-nitroso-1,4-benzoxazin als Öl erhalten.

F) 8 g der vorstehend erhaltenen Nitrosoverbindung wurden nach der in Beispiel 1D) beschriebenen Methode reduziert. Es wurden 4 g 4-Amino-2,3-dihydro-2-(2-hydroxyethyl)-6-methoxy-1,4-benzoxazin als klebrige Verbindung erhalten.

G) 4 g der vorstehend erhaltenen Hydrazinverbindung wurden mit 4-Hydroxypropiophenon nach der in Beispiel 1E) beschriebenen Methode umgesetzt. Es wurden 2,4 g 2,3-Dihydro-2-(2-hydroxethyl)-5-(4-hydroxyphenyl)-7-methoxy-6-methyl-pyrrolo[1,2,3-de]-1,4-benzoxazin als weißer Feststoff mit einem Schmelzpunkt von 190 °C erhalten.

H) 2,4 g des vorstehend erhaltenen Alkohols wurden nach der in Beispiel 1F) beschriebenen Methode in das entsprechende Tosylat überführt. Es wurden 2,3 g 2,3-Dihydro-5-(4-hydroxyphenyl)-7-methoxy-6-methyl-2-(2-(p-toluolsulfonyloxy)-ethyl)-pyrrolo[1,2,3-de]-1,4-benzoxazin als Öl erhalten.

I) 2,3 g des vorstehend erhaltenen Produktes wurden nach der in Beispiel 1G) beschriebenen Methode mit 1-[2-(4-Methylpyridyl)]piperazin umgesetzt. Die erhaltene rohe Titelverbindung wurde aus Diethylether kristallisiert. Es wurden 0,3 g der Titelverbindung als gelbes Pulver mit einem Schmelzpunkt von 193 bis 195 °C erhalten.

**Beispiel 4:**

2,3-Dihydro-7-hydroxy-5-(4-hydroxyphenyl)-6-methyl-2-[2-(4-(4-methylpyridin-2-yl)-piperazin-1-yl)-ethyl]-pyrrolo[1,2,3-de]-1,4-benzoxazin.

0,1 g 2,3-Dihydro-5-(4-hydroxyphenyl)-7-methoxy-6-methyl-2-[2-(4-(4-methylpyridin-2-yl)-piperazin-1-yl)-ethyl]-pyrrolo[1,2,3-de]-1,4-benzoxazin (Herstellung siehe Beispiel 3)) wurden nach der in Beispiel 2 beschriebenen Methode durch Behandlung mit Bortribromid demethyliert. Es wurden 0,03 g der Titelverbindung als weißes Pulver mit einem Schmelzpunkt von 250 °C (Zersetzung) erhalten.

**Beispiel 5:**

2,3-Dihydro-5-(4-methoxyphenyl)-6-methyl-2-[4-(4-methylpyridin-2-yl)-piperazin-1-ylmethyl]-pyrrolo[1,2,3-de]-1,4-benzoxazin.

A) 68 g 2-Aminophenol wurden zu einer Mischung aus 430 g Kaliumcarbonat und 1 l Dimethylformamid gegeben. Das Reaktionsgemisch wurde eine halbe Stunde lang bei Raumtemperatur gerührt. Dann wurden 208 g 2,3-Dibrompropionsäureethylester tropfenweise zugegeben und anschließend wurde das Reaktionsgemisch für weitere 12 Stunden bei einer Temperatur von 45 °C gerührt. Zur Aufarbeitung wurde das Reaktionsgemisch filtriert und das Filtrat in 2 l Wasser gegossen. Das Gemisch wurde mit dreimal 800 ml Ethylacetat extrahiert und die organischen Extrakte wurden getrocknet, filtriert und eingedampft. Es wurden 110 g 4H-2,3-Dihydro-1,4-benzoxazin-2-carbonsäureethylester als Öl erhalten.

B) 100 g des vorstehend erhaltenen Esters wurden analog der in Beispiel 1B) beschriebenen Methode mit Lithiumaluminiumhydrid in Tetrahydrofuran reduziert. Es wurden 56 g 4H-2,3-Dihydro-2-hydroxymethyl-1,4-benzoxazin als Öl erhalten.

C) 56 g des vorstehend erhaltenen Produktes wurden nach der in Beispiel 1C) beschriebenen Methode nitrosiert. Es wurden 40 g 2,3-Dihydro-2-hydroxymethyl-4-nitroso-1,4-benzoxazin als Öl erhalten.

D) 40 g der vorstehend erhaltenen Nitrosoverbindung wurden nach der in Beispiel 1D) beschriebenen Methode mit Lithiumaluminumhydrid in Tetrahydrofuran reduziert. Es wurden 24,5 g 4-Amino-2,3-dihydro-2-hydroxymethyl-1,4-benzoxazin als klebrige Verbindung erhalten.

E) 24,5 g der vorstehend erhaltenen Hydrazinverbindung wurden nach der in Beispiel 1E) beschriebenen Methode mit 4-Methoxypropiophenon umgesetzt. Es wurden 34 g 2,3-Dihydro-2-hydroxymethyl-5-(4-methoxyphenyl)-6-methyl-pyrrolo[1,2,3-de]-1,4-benzoxazin als Öl erhalten.

F) 34 g des vorstehend erhaltenen Alkohols wurden nach der in Beispiel 1F) beschriebenen Methode in das entsprechende Tosylat überführt. Es wurden 50 g 2,3-Dihydro-5-(4-methoxyphenyl)-6-methyl-2-(p-toluolsulfonyloxymethyl)-pyrrolo[1,2,3-de]-1,4-benzoxazin als Öl erhalten.

G) 12 g der vorstehend erhaltenen Tosylatverbindung wurden nach der in Beispiel 1G) beschriebenen Methode mit 1-(4-Methylpyridin-2-yl)-piperazin umgesetzt. Es wurden 5 g der Titelverbindung als weißes Pulver mit einem Schmelzpunkt von 260 °C erhalten.

**Beispiel 6:**

2,3-Dihydro-5-(4-methoxyphenyl)-6-methyl-2-[3-(4-(4-methylpyridin-2-yl)-piperazin-1-yl)-3-oxopropyl]-pyrrolo[1,2,3-de]-1,4-benzoxazin.

A) 13,7 g 2,3-Dihydro-2-[2-(p-toluolsulfonyloxy)-ethyl]-5-(4-methoxyphenyl)-6-methyl-pyrrolo[1,2,3-de]-1,4-benzoxazin (Herstellung siehe Beispiel 1F)) wurden in 50 ml heißem Dimethylsulfoxid unter Stickstoffatmosphäre gelöst. Zu dem auf 80 °C erhitzten Reaktionsgemisch wurden 2,2 g Kaliumcyanid gegeben und das Gemisch wurde weitere 6 Stunden gerührt. Zur Aufarbeitung wurde das Reaktionsgemisch nach Abkühlen in Eiswasser gegossen. Der sich bildende braune Feststoff wurde filtriert und in Dichlormethan gelöst. Die organische Lösung wurde mit Wasser gewaschen, getrocknet und eingedampft. Das als Rückstand verbleibende Rohprodukt wurde durch Säulenchromatographie unter Verwendung von Dichlormethan als Elutionsmittel gereinigt. Es wurden 9,1 g 2,3-Dihydro-2-(2-cyanoethyl)-5-(4-methoxyphenyl)-6-methylpyrrolo[1,2,3-de]-1,4-benzoxazin als weißer Feststoff mit einem Schmelzpunkt von 113 bis 114 °C erhalten.

B) In eine Lösung von 1,9 g der vorstehend erhaltenen Nitrilverbindung in 15 ml Ethanol wurde gasförmiger Chlorwasserstoff geleitet und das Reaktionsgemisch wurde 2 Stunden am Rückfluß gekocht. Dann wurden 3 ml Wasser zugegeben und weitere 5 Stunden unter Rühren am Rückfluß erhitzt. Zur Aufarbeitung wurden dem Gemisch nach Abkühlung auf Raumtemperatur 50 ml Wasser zugegeben. Das Reaktionsprodukt wurde mit Dichlormethan extrahiert und durch Säulenchromatographie unter Verwendung von Petrolether/Diethylether 70:30 als Elutionsmittel gereinigt. Es wurden 2,2 g 3-[2,3-Dihydro-5-(4-methoxyphenyl)-6-methyl-pyrrolo[1,2,3-de]-1,4-benzoxazin-2-yl]-propionsäureethylester als weißer Feststoff mit einem Schmelzpunkt von 154 °C erhalten.

C) Zu einer Lösung von 2,2 g des vorstehend erhaltenen Esters in 10 ml Ethanol wurde eine Lösung von 3,5 g Kaliumhydroxid in 2 ml Wasser gegeben. Das Reaktionsgemisch wurde 1 Stunde am Rückfluß erhitzt. Dann wurde das Reaktionsgemisch auf Raumtemperatur abgekühlt und mit 20 ml Wasser verdünnt. Die erhaltene Lösung wurde durch Zugabe von 10%iger Salzsäurelösung angesäuert und mit Dichlormethan extrahiert. Die organische Phase wurde über Natriumsulfat getrocknet und eingedampft. Es wurden 1,6 g 3-[2,3-Dihydro-5-(4-methoxyphenyl)-6-methyl-pyrrolo[1,2,3-de]-1,4-benzoxazin-2-yl]-propionsäure als weißer Feststoff erhalten.

D) 1,6 g der vorstehend erhaltenen Säure wurden unter Stickstoffatmosphäre in 10 ml wasserfreiem Dichlormethan gelöst. Zu der Lösung wurden 0,73 g Carbonyldiimidazol gegeben und das Reaktionsgemisch wurde dann 1 Stunde bei Raumtemperatur gerührt. Anschließend wurde 1 g 1-(4-Methylpyrid-2-yl)-piperazin zugegeben und das Reaktionsgemisch wurde 1 Stunde am Rückfluß erhitzt. Dann wurde das Reaktionsgemisch auf Raumtemperatur abgekühlt und mit alkalisch gestelltem Wasser (pH = 12) gewaschen. Anschließend wurde die organische Phase getrocknet, filtriert und eingeengt. Der verbleibende Rückstand wurde durch Säulenchromatographie gereinigt (Elutionsmittel Dichlormethan enthaltend von 1 bis 8 Vol.-% ansteigende Mengen Ethanol). Es wurden 2 g 2,3-Dihydro-5-(4-methoxyphenyl)-6-methyl-2-[3-(4-(4-methyl-pyridin-2-yl)-piperazin-1-yl)-3-oxopropyl]-pyrrolo[1,2,3-de]-1,4-benzoxazin • 0,2 $H_2O$ als weiße Kristalle mit einem Schmelzpunkt von 82 bis 83 °C erhalten.

**Beispiel 7:**

2,3-Dihydro-5-(4-methoxyphenyl)-6-methyl-2-[3-(4-(4-methylpyridin-2-yl)-piperazin-1-yl)-propyl]-pyrrolo-[1,2,3-de]-1,4-benzoxazin.

1 g 2,3-Dihydro-5-(4-methoxyphenyl)-6-methyl-2-[3-(4-(4-methylpyridin-2-yl)-piperazin-1-yl)-3-oxopropyl]-pyrrolo[1,2,3-de]-1,4-benzoxazin (Herstellung siehe Beispiel 6)) wurde unter Stickstoffatmosphäre in 15 ml wasserfreiem Tetrahydrofuran gelöst. Zu der Lösung wurden 12 ml eines 1-molaren Boran-tetrahydrofurankomplexes gegeben. Das Reaktionsgemisch wurde gerührt und 2 Stunden am Rückfluß erhitzt. Dann wurden 25 ml einer 6 N Salzsäurelösung zugegeben und das Reaktionsgemisch 24 Stunden gerührt. Der gebildete Niederschlag wurde in alkalisch gestelltem Wasser (pH = 12) gelöst und mit Dichlormethan extrahiert. Die organischen Extrakte wurden getrocknet und eingedampft. Das verbleibende Rohprodukt wurde durch Säulenchromatographie unter Verwendung von 2 % Ethanol enthaltendem Dichlormethan als

Elutionsmittel gereinigt. Die gereinigte Titelverbindung wurde zur Überführung in ihr Dihydrochlorid-Salz in 3 N isopropanolische Salzsäurelösung gegeben. Es wurden 0,6 g 2,3-Dihydro-5-(4-methoxyphenyl)-6-methyl-2-[3-(4-(4-methylpyridin-2-yl)-piperazin-1-yl)-propyl]-pyrrolo[1,2,3-de]-1,4-benzoxazin•2HCl•0,7$H_2O$ als gelbe Kristalle mit einem Schmelzpunkt von 248 bis 249 °C erhalten.

**Beispiel 8:**

2,3-Dihydro-5-(4-methoxyphenyl)-6-methyl-2-[2-(4-(4-methylpyridin-2-yl)-piperazin-1-yl)-2-oxoethyl]-pyrrolo-[1,2,3-de]-1,4-benzoxazin.

A) 92 g (3-Oxo-2,3-dihydro-4H-1,4-benzoxazin-2-yl)-essigsäuremethylester (Herstellung siehe Beispiel 1A)) wurden in 300 ml Dichlormethan suspendiert. Zu der Suspension wurden 430 ml einer 1-molaren Lösung eines Boran-tetrahydrofurankomplexes in Tetrahydrofuran gegeben. Das Reaktionsgemisch wurde 48 Stunden bei Raumtemperatur gerührt. Dann wurden 250 ml 35%iger Salzsäurelösung zu dem Reaktionsgemisch gegeben und weitere 2 Stunden gerührt. Das Reaktionsgemisch wurde durch Zugabe von 10%iger Ammoniaklösung neutralisiert und mit Dichlormethan extrahiert. Die organischen Extrakte wurden mit Wasser gewaschen, getrocknet, filtriert und eingeengt. Das als Rückstand verbleibende Öl wurde in 500 ml Aceton gelöst. Durch Zugabe von isopropanolischer Salzsäurelösung und Kühlen auf +5 °C wurde aus der Lösung (2,3-Dihydro-4H-1,4-benzoxazin-2-yl)-essigsäuremethylester-hydrochlorid gefällt. Es wurden 70 g des Hydrochlorids als kristalliner Feststoff erhalten.

B) 70 g der vorstehenden Verbindung wurden nach der in Beispiel 1C) beschriebenen Methode nitrosiert. Es wurden 65 g (2,3-Dihydro-4-nitroso-1,4-benzoxazin-2-yl)-essigsäuremethylester als Öl erhalten.

C) Zu einer Suspension von 60 g Zink in 150 ml Wasser wurde eine Lösung von 60 g der vorstehend erhaltenen Nitrosoverbindung in 700 ml Essigsäure gegeben und das Reaktionsgemisch wurde 12 Stunden gerührt. Anschließend wurde eingedampft und der verbleibende Rückstand wurde in 1 l Dichlormethan gelöst. Die organische Lösung wurde zweimal mit 500 ml alkalisch gestelltem Wasser (pH = 9) und anschließend zweimal mit 1 l neutralem Wasser gewaschen, über Natriumsulfat getrocknet, filtriert und eingeengt. Das verbleibende Rohprodukt wurde durch Säulenchromatographie unter Verwendung von von 1 bis 10 Vol.-% ansteigende Mengen Methanol enthaltendem Dichlormethan als Elutionsmittel gereinigt. Es wurden 25 g (4-Amino-2,3-dihydro-1,4-benzoxazin-2-yl)-essigsäuremethylester als Öl erhalten.

D) 25 g der vorstehend erhaltenen Hydrazin-Verbindung wurden nach der in Beispiel 1E) beschriebenen Methode mit 4-Methoxypropiophenon umgesetzt, wobei als Reaktionsmedium Methanol verwendet wurde. Das Reaktionsgemisch wurde wie in Beispiel 1E) beschrieben aufgearbeitet. Es wurden 10 g (2,3-Dihydro-5-(4-methoxyphenyl)-6-methyl-pyrrolo[1,2,3-de]-1,4-benzoxazin-2-yl)-essigsäuremethylester als Öl erhalten.

E) 10 g des vorstehend erhaltenen Esters wurden nach der in Beispiel 6C) beschriebenen Methode hydrolysiert. Die erhaltene Säure wurde durch Säulenchromatographie unter Verwendung von von 0 bis 10 Vol.- % ansteigende Mengen Methanol enthaltendem Ethylacetat als Elutionsmittel gereinigt. Es wurden 3,2 g (2,3-Dihydro-5-(4-methoxyphenyl)-6-methyl-pyrrolo[1,2,3-de]-1,4-benzoxazin-2-yl)-essigsäure als klebriger Rückstand erhalten.

F) 3,2 g der vorstehend erhaltenen Säure wurden nach der in Beispiel 6D) beschriebenen Methode mit 3 g 1-(4-Methylpyridin-2-yl)]-piperazin umgesetzt. Die erhaltene rohe Titelverbindung wurde durch Säulenchromatographie unter Verwendung von von 0 bis 10 Vol.-% ansteigende Mengen Methanol enthaltendem Ethylacetat gereinigt. Es wurden 1,7 g der Titelverbindung als Öl erhalten. NMR-Spektrum (60 MHz, s = Singlett, d = dublett, m = multiplett, br = breit): 7,9 ppm (d,1H), 7,4 - 6,1 ppm (m,9H), 4,7 ppm (br s,3H), 3,8 ppm (s,3H), 3,6 - 3 ppm (m,8H), 2,7 - 2,5 ppm (m,2H), 2,3 - 2 ppm (2s,6H)

**Beispiel 9:**

2,3-Dihydro-5-(3,5-dimethyl-4-hydroxyphenyl)-6-methyl-2-[4-(4-(4-methylpyridin-2-yl)-piperazin-1-yl)-4-oxobutyl]-pyrrolo[1,2,3-de]-1,4-benzoxazin.

A) Ein Gemisch aus 15,4 g 2-Nitrophenol, 31 g 2-Bromadipinsäuredimethylester (hergestellt durch Bromieren von Adepinsäuredimethylester mit N-Bromsuccinimid in Tetrachlormethan in Gegenwart von Benzoylperoxid nach der in J. Org. Chem. 18 (1953) 649 bis 652 beschriebenen Methode.) und 46 g Kaliumcarbonat in 100 ml Dimethylformamid wurde unter Rühren 4 Stunden auf 40 °C erhitzt. Zur Aufarbeitung wurde das Reaktionsgemisch filtriert, das Filtrat in 200 ml Wasser gegeben und das

Reaktionsprodukt mit dreimal je 100 ml Ethylacetat extrahiert und durch Säulenchromatographie unter Verwendung von Dichlormethan als Elutionsmittel gereinigt. Es wurden 28 g 2-(2-Nitrophenoxy)-adipinsäuredimethylester als Öl erhalten.

B) Eine Lösung von 13,5 g des vorstehend erhaltenen Produktes in 300 ml Methanol wurde mit 0,7 g eines Palladium/Kohle-Katalysators (10 % Palladium auf Kohle) versetzt. Dann wurde bei Raumtemperatur mit einem Wasserstoffdruck von 4 bar während 5 Stunden hydriert. Anschließend wurde das Reaktionsgemisch vom Katalysator abfiltriert und eingedampft. Es wurden 9 g 4-(3-Oxo-2,3-dihydro-4H-1,4-benzoxazin-2-yl)-buttersäuremethylester als Öl erhalten.

C) 11,5 g des vorstehend erhaltenen Produktes wurden nach der in Beispiel 8A) beschriebenen Methode mit einem Boran-tetrahydrofurankomplex reduziert. Es wurden 5 g 4-(2,3-Dihydro-4H-1,4-benzoxazin-2-yl)-buttersäuremethylester als Öl erhalten.

D) 5 g des vorstehend erhaltenen Produktes wurden nach der in Beispiel 1C) beschriebenen Methode durch Umsetzen mit Natriumnitrit nitrosiert. Es wurden 5 g 4-(2,3-Dihydro-4-nitroso-1,4-benzoxazin-2-yl)-buttersäuremethylester als Öl erhalten.

E) 5 g des vorstehend erhaltenen Nitrosoproduktes wurden nach der in Beispiel 8C) beschriebenen Methode mit Zink/Essigsäure reduziert. Es wurden 4,5 g 4-(4-Amino-2,3-dihydro-1,4-benzoxazin-2-yl)-buttersäuremethylester als klebrige Verbindung erhalten.

F) 4,5 g des vorstehend erhaltenen Produktes wurden mit (3,5-Dimethyl-4-hydroxy)propiophenon (hergestellt nach der in Bull. Soc. Chim. Fra., 1977, 901 bis 905 von R. Martin beschriebenen Methode durch Umsetzung von 2,6-Dimethylphenol mit Propionsäurechlorid in Dichlormethan in Gegenwart von Triethylamin und anschließendes Behandeln des gebildeten 2,6-Dimethylphenol-propionsäureesters mit Aluminiumtrichlorid in Nitromethan) nach der in Beispiel 1E) beschriebenen Methode umgesetzt. Es wurden 5,2 g 4-[2,3-Dihydro-5-(3,5-dimethyl-4-hydroxyphenyl)-6-methyl-pyrrolo[1,2,3-de]-1,4-benzoXazin-2-yl]-buttersäuremethylester als Öl erhalten.

G) 5,2 g des vorstehend erhaltenen Esters wurden nach der in Beispiel 6C) beschriebenen Methode hydrolisiert. Es wurden 2 g 4-[2,3-Dihydro-5-(3,5-dimethyl-4-hydroxyphenyl)-6-methyl-pyrrolo[1,2,3-de]-1,4-benzoxazin-2-yl]-buttersäure als klebriges Produkt erhalten.

H) 2 g der vorstehend erhaltenen Säure wurden mit 1-(4-Methylpyridin-2-yl)-piperazin nach der in Beispiel 6D) beschriebenen Methode in Dichlormethan in Gegenwart von Carbonyldiimidazol umgesetzt. Es wurden 0,8 g der Titelverbindung als weiße Kristalle mit einem Schmelzpunkt von 112 °C erhalten.

**Beispiel 10:**

2,3-Dihydro-5-(4-methoxyphenyl)-2-[2-(4-(4-methylpyridin-2-yl)-piperazin-1-yl)-ethyl]-pyrrolo[1,2,3-de]-1,4-benzoxazin.

A) Eine Lösung von 7,2 g Methoxybenzol in 120 ml Dichlormethan wurde in einem Eisbad auf 0 °C abgekühlt. Dann wurden langsam 22 g Aluminiumtrichlorid zugegeben. Die Farbe der Lösung änderte sich dabei von gelb auf rosa. Anschließend wurden 10 g Malonsäuremonoethylesterchlorid zugegeben, wobei die Farbe der Lösung sich von rosa nach braun veränderte. Das Reaktionsgemisch wurde eine halbe Stunde unter Rückfluß erhitzt und dann in eine Eis/Wassermischung gegeben und mit Dichlormethan extrahiert. Die organischen Extrakte wurden mit Wasser gewaschen, getrocknet, filtriert und eingedampft. Es wurden 4,5 g 3-(4-Methoxyphenyl)-3-oxopropionsäureethylester als Öl erhalten.

B) 9 g des vorstehend erhaltenen Esters wurden mit 8 g 4-Amino-2,3-dihydro-2-(2-hydroxyethyl)-1,4-benzoxazin (Herstellung siehe Beispiel 1D)) nach der in Beispiel 1E) beschriebenen Methode umgesetzt. Das erhaltene Rohprodukt wurde durch Säulenchromatographie unter Verwendung von 1 % Ethanol enthaltendem Dichlormethan als Elutionsmittel gereinigt. Es wurden 6 g 2,3-Dihydro-2-(2-hydroxyethyl-5-(4-methoxyphenyl)-pyrrolo[1,2,3-de]-1,4-benzoxazin-6-carbonsäureethylester als Öl erhalten.

C) 6 g der vorstehend erhaltenen Verbindung wurden nach der in Beispiel 1F) beschriebenen Methode mit p-Toluolsulfonsäurechlorid in Pyridin umgesetzt. Es wurden 8 g rohes 2,3-Dihydro-5-(4-methoxyphenyl)-2-[2-(p-toluolsulfonyloxy)ethyl]-pyrrolo[1,2,3-de]-1,4-benzoxazin-6-carbonsäureethylester als klebriger Feststoff erhalten, welcher ohne weitere Reinigung weiterverarbeitet wurde.

D) 8 g des vorstehend erhaltenen Tosylates wurden nach der in Beispiel 1G) beschriebenen Methode mit 4 g 1-(4-Methylpyridin-2-yl)-piperazin in Dimethylformamid umgesetzt. Das erhaltene Reaktionsprodukt wurde durch Kristallisation aus Diethylether gereinigt. Es wurden 1,8 g 2,3-Dihydro-5-(4-methoxyphenyl)-2-[2-(4-(4-methylpyridin-2-yl)-piperazin-1-yl)-ethyl]-pyrrolo[1,2,3-de]-1,4-benzoxazin-6-carbonsäureethylester als weißes Pulver mit einem Schmelzpunkt von 149 bis 150 °C erhalten.

E) 0,5 g des vorstehend erhaltenen Esters wurden mit 0,1 g Kaliumhydroxid in 10 ml Ethanol 3 Stunden unter Rückfluß erhitzt. Anschließend wurde das Lösungsmittel abgedampft und der verbleibende Rückstand wurde 8 Stunden auf 170 °C erhitzt. Dann wurde der Rückstand in 20 ml Ethylacetat gelöst, die Lösung mit zweimal je 10 ml Wasser gewaschen und durch Säulenchromatographie gereinigt. Die so erhaltene Titelverbindung wurde durch Behandeln mit 3 N isopropanolischer Salzsäure in ihr Dihydrochlorid überführt. Es wurden 0,3 g 2,3-Dihydro-5-(4-methoxyphenyl)-2-[2-(4-(4-methylpyridin-2-yl)-piperazin-1-yl)-ethyl]-pyrrolo[1,2,3-de]-1,4-benzoxazin•2HCl•1,5$H_2O$ als braunes Pulver mit einem Schmelzpunkt von 200 °C (Zersetzung) erhalten.

**Beispiel 11:**

2,3-Dihydro-5-(3,5-dimethyl-4-hydroxyphenyl)-6-methyl-2-[3-(4-(4-methylpyridin-2-yl)-piperazin-1-yl)-propyl]-1,4-benzoxazin.

A) 24 g 2-Aminophenol, 55 g 2,5-Dibromvaleriansäuremethylester und 30 g Kaliumcarbonat wurden in 250 ml Aceton 5 Stunden am Rückfluß erhitzt. Zur Aufarbeitung wurde das Reaktionsgemisch mit 250 ml Diethylether verdünnt, mit 200 ml Wasser gewaschen und mit 12 N Salzsäurelösung bis auf pH = 1 angesäuert. Die organische Phase wurde abgetrennt, mit zweimal je 100 ml Wasser gewaschen, getrocknet und eingedampft. Der Rückstand wurde aus Methanol kristallisiert. Es wurden 11 g 2-(3-Brompropyl)-2H-1,4-benzoxazin-3(4H)-on erhalten.

B) 8,5 g des vorstehend erhaltenen Produktes wurden mit 5,6 g 1-(4-Methylpyridin-2-yl)-piperazin und 3,5 g Triethylamin in 80 ml Toluol 1 Stunde am Rückfluß gekocht. Zur Aufarbeitung wurde das Reaktionsgemisch mit zweimal 50 ml Wasser gewaschen und eingeengt. Der verbleibende Rückstand wurde durch Säulenchromatographie unter Verwendung von Ethylacetat als Elutionsmittel gereinigt. Es wurden 10 g 2-[3-(4-(4-Methylpyridin-2-yl)-piperazin-1-yl)-propyl]-2H-1,4-benzoxazin-3(4H)-on als klebrige Verbindung erhalten.

C) 10 g der vorstehend erhaltenen Verbindung wurden nach der in Beispiel 1B) beschriebenen Methode mit Lithiumaluminiumhydrid in Tetrahydrofuran reduziert. Es wurden 9 g 4H-2,3-Dihydro-2-[3-(4-(4-methylpyridin-2-yl)-piperazin-1-yl)-propyl]-1,4-benzoxazin als Öl erhalten.

D) 9 g der vorstehend erhaltenen Verbindung wurden durch Umsetzung mit Natriumnitrit nach der in Beispiel 1C) beschriebenen Methode nitrosiert. Es wurden 8 g 2,3-Dihydro-2-[3-(4-(4-methylpyridin-2-yl)-piperazin-1-yl)-propyl]-4-nitroso-1,4-benzoxazin als Öl erhalten.

E) 8 g der vorstehend erhaltenen Nitrosoverbindung wurden nach der in Beispiel 1D) beschriebenen Methode mit Lithiumaluminiumhydrid in Tetrahydrofuran reduziert. Es wurden 6 g 4-Amino-2,3-dihydro-2-[3-(4-(4-methylpyridin-2-yl)-piperazin-1-yl)-propyl]-1,4-benzoxazin erhalten.

F) 6 g des vorstehend erhaltenen Produktes und 3 g (3,5-Dimethyl-4-hydroxy)-4-propiophenon wurden in 60 ml Ethanol gelöst. Das Reaktionsgemisch wurde 2,5 Stunden unter Stickstoffatmosphäre am Rückfluß erhitzt. Dann wurden bei der gleichen Temperatur 10 ml 12 N Salzsäurelösung tropfenweise über einen Zeitraum von einer Stunde verteilt zugegeben. Zur Aufarbeitung wurde das Reaktionsgemisch mit Wasser verdünnt und mit Dichlormethan extrahiert. Die organische Phase wurde über Natriumsulfat getrocknet, filtriert und eingeengt. Das erhaltene Rohprodukt wurde aus einem Gemisch Diethylether/n-Hexan 1:1 kristallisiert. Es wurden 5 g der Titelverbindung als weißes Pulver mit einem Schmelzpunkt von 110 °C erhalten.

Nach den in den vorstehenden Beispielen beschriebenen Verfahren können auch die in der nachfolgenden Tabelle 1 aufgeführten Verbindungen der Formel I hergestellt werden.

**Tabelle 1**

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 12 | $CH_3$ | H | H | $CH_3O$ | H | H | O | 2 | bi | N | 4-$CH_3$-pyrid-2- | BA (0,6) | 88-89 |
| 13 | $CH_3$ | H | H | HO | H | H | O | 2 | bi | N | 4-$CH_3$-pyrid-2- | BA (0,7) | 253-254 |
| 14 | $CH_3$ | H | H | H | H | H | O | 1 | bi | N | 4-$CH_3$-pyrid-2- | 2,3 HCl (2) | 218-220 |
| 15 | $CH_3$ | H | H | H | H | H | O | 2 | CO | N | 4-$CH_3$-pyrid-2- | BA (3,1) | 152-153 |
| 16 | $CH_3$ | H | H | H | H | H | O | 2 | $CH_2$ | N | 4-$CH_3$-pyrid-2- | BA (0,7) | 200-201 |
| 17 | $CH_3$ | H | H | H | H | H | O | 1 | CO | N | 4-$CH_3$-pyrid-2- | BA | 150 (Z) |
| 18 | $CH_3$ | H | H | $CH_3$ | $CH_3$ | H | O | 3 | $CH_2$ | N | 4-$CH_3$-pyrid-2- | BA (0,6) | 70-71 |
| 19 | $CH_3$ | 7-Cl | H | H | H | H | O | 2 | bi | N | 4-$CH_3$-pyrid-2- | 2 HCl (1,5) | 220 (Z) |
| 20 | $CH_3$ | H | H | $CH_3$ | $CH_3$ | H | O | 2 | bi | N | 4-$CH_3$-pyrid-2- | 2 HCl (2,5) | 190-192 |
| 21 | $CH_3$ | H | H | H | H | H | S | 2 | bi | N | 4-$CH_3$-pyrid-2- | 2 HCl (3) | 204-206 |

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 22 | $CH_3$ | 7-F | H | H | H | H | O | 2 | bi | N | 4-$CH_3$-pyrid-2- | BA (0,6) | 128-130 |
| 23 | $CH_3$ | H | H | $CH_3$ | $CH_3$ | H | O | 3 | CO | N | pyrid-2- | BA | 208 |
| 24 | $CH_3$ | H | H | H | H | H | O | 2 | bi | N | 4-F-phen- | HCl (3) | 171-173 |
| 25 | $CH_3$ | H | H | $CH_3$ | $CH_3$ | H | O | 2 | CO | N | 4-$CH_3$-pyrid-2- | BA | 184-185 |
| 26 | $CH_3$ | H | H | $CH_3$ | $CH_3$ | H | O | 2 | CO | N | pyrid-2- | HCl (1) | 180-182 |
| 27 | $CH_3$ | 7-F | H | $CH_3$ | $CH_3$ | H | O | 2 | bi | N | pyrid-2- | 2 HCl (2,2) | 225 (Z) |
| 28 | $CH_3$ | 7-F | H | $CH_3$ | $CH_3$ | H | O | 2 | bi | N | 4-$CH_3$-pyrid-2- | 2,3 HCl (5) | 225 (Z) |
| 29 | $CH_3$ | 7-F | H | $CH_3$ | $CH_3$ | H | O | 2 | $CH_2$ | N | 4-$CH_3$-pyrid-2- | 2 HCl (3) | 267-269 |
| 30 | $CH_3$ | H | H | H | H | H | O | 1 | $CH_2$ | N | pyrid-2- | BA (0,2) | 199-201 |
| 31 | $CH_3$ | H | H | H | H | H | O | 2 | bi | N | 5-$CH_3$-pyrid-2- | BA (0,5) | 217-220 |
| 32 | $CH_3$ | H | H | H | H | H | O | 2 | bi | N | 3-$CH_3$-pyrid-2- | BA (0,2) | 189-191 |

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 33 | $CH_3$ | H | H | H | H | H | O | 2 | bi | N | 6-$CH_3$-pyrid-2- | BA (1,1) | 176-178 |
| 34 | $CH_3$ | H | H | H | H | H | O | 2 | CO | N | 5-$CH_3$-pyrid-2- | HCl (3) | 243-245 |
| 35 | $CH_3$ | H | H | H | $CH_3O$ | H | O | 2 | bi | N | 3-$CH_3$-pyrid-2- | 2,5 HCl (3,7) | 230-231 |
| 36 | $CH_3$ | H | H | H | H | H | O | 2 | CO | N | pyrid-2- | BA (1,5) | 120-122 |
| 37 | $CH_3$ | H | H | H | H | H | O | 2 | CO | N | 3-$CH_3$-pyrid-2- | BA (0,2) | 112-114 |
| 38 | $CH_3$ | H | H | H | $CH_3O$ | H | O | 2 | CO | N | 4-$CH_3$-pyrid-2- | BA | 114-116 |
| 39 | $CH_3$ | H | H | H | $CH_3O$ | H | O | 2 | CO | N | 5-$CH_3$-pyrid-2- | BA (1,1) | 95-97 |
| 40 | $CH_3$ | H | H | $CH_3$ | $CH_3$ | H | O | 1 | $CH_2$ | CH | opyrim-N-<br>$CH_3$ | BA (1) | 255-257 |
| 41 | $CH_3$ | H | H | H | H | H | O | 2 | $CH_2$ | N | 3-$CH_3$-pyrid-2- | BA | 116-118 |
| 42 | $CH_3$ | 8-F | H | $CH_3$ | $CH_3$ | H | O | 2 | bi | N | 4-$CH_3$-pyrid-2- | BA | 108 |
| 43 | $CH_3$ | 8-$CH_3O$ | H | $CH_3$ | $CH_3$ | H | O | 2 | bi | N | 4-$CH_3$-pyrid-2- | BA | 100 |

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 44 | $CH_3$ | 8-HO | H | $CH_3$ | $CH_3$ | H | O | 2 | bi | N | 4-$CH_3$-pyrid-2- | BA (1,1) | 195 |
| 45 | $CH_3$ | 7-Cl | H | H | H | $CH_3$ | O | 2 | bi | N | 4-$CH_3$-pyrid-2- | BA | 178 |
| 46 | $CH_3$ | 7-Cl | H | $CH_3$ | $CH_3$ | $CH_3$ | O | 2 | bi | N | 4-$CH_3$-pyrid-2- | 2,5 HCl (4) | 250 (Z) |
| 47 | $CH_3$ | H | H | $CH_3$ | $CH_3$ | $CH_3$ | O | 2 | bi | N | 4-$CH_3$-pyrid-2- | 2 HCl (3) | 169 |
| 48 | $CH_3$ | 7-Cl | H | $CH_3$ | $CH_3$ | H | O | 2 | bi | N | 4-$CH_3$-pyrid-2- | 2 HCl (1) | 250 (Z) |
| 49 | $CH_3$ | H | H | HO | H | H | O | 2 | CO | N | 4-$CH_3$-pyrid-2- | BA (1,7) | 120 |
| 50 | $CH_3$ | H | H | H | H | $CH_3$ | S | 2 | bi | N | 4-$CH_3$-pyrid-2- | 2 HCl (2,5) | 195 |
| 51 | $CH_3$ | 7-F | H | H | H | $CH_3$ | O | 2 | bi | N | 4-$CH_3$-pyrid-2- | 2 HCl (3) | 275 (Z) |
| 52 | $CH_3$ | H | H | HO | H | $CH_3$ | O | 2 | bi | N | 4-$CH_3$-pyrid-2- | BA | 121-122 |
| 53 | $CH_3$ | H | H | H | H | $CH_3$ | O | 2 | bi | N | 4-F-phen- | HCl (2) | 210-214 |
| 54 | $CH_3$ | 7-F | H | $CH_3$ | $CH_3$ | H | O | 2 | CO | N | 4-$CH_3$-pyrid-2- | BA (1,5) | 168-170 |

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 55 | $CH_3$ | H | H | H | H | H | O | 2 | $CH_2$ | N | 5-$CH_3$-pyrid-2- | 2 HCl (2,1) | 247-249 |
| 56 | $CH_3$ | H | H | H | H | H | O | 2 | $CH_2$ | N | pyrid-2- | 2 HCl (2,4) | 257-260 |
| 57 | $CH_3$ | H | H | H | H | H | O | 2 | CO | N | 6-$CH_3$-pyrid-2- | 1,1 HCl (2,8) | 237-240 |
| 58 | $CH_3$ | H | H | H | H | H | O | 3 | bi | N | 6-$CH_3$-pyrid-2- | 2,2 HCl (3) | 257-259 |
| 59 | $CH_3$ | H | H | $CH_3$ | $CH_3$ | H | O | 2 | bi | N | pyrid-2- | 2 HCl (2,5) | 140 (Z) |
| 60 | $CH_3$ | H | H | $CH_3$ | $CH_3$ | H | O | 2 | bi | N | 3-$CH_3$-pyrid-2- | 2 HCl (2) | 170 (Z) |
| 61 | $CH_3$ | H | H | $CH_3$ | $CH_3$ | H | O | 2 | bi | N | 6-$CH_3$-pyrid-2- | 2 HCl (2,2) | 140 (Z) |
| 62 | $CH_3$ | H | H | $CH_3$ | $CH_3$ | H | O | 2 | bi | N | 5-$CH_3$-pyrid-2- | 2 HCl (2,8) | 100 (Z) |
| 63 | $CH_3$ | H | H | $CH_3$ | $CH_3$ | H | S | 3 | $CH_2$ | N | 4-$CH_3$-pyrid-2- | BA | 84-85 |
| 64 | $CH_3$ | H | H | $OCH_3$ | $CH_3$ | H | S | 3 | $CH_2$ | N | 4-$CH_3$-pyrid-2- | BA | 85-86 |
| 65 | $CH_3$ | H | H | $CH_3$ | $CH_3$ | H | O | 3 | $CH_2$ | N | pyrid-2- | BA (1) | 72-73 |

| Bsp. Nr. | $R^1$ | $R^2$ | $R^3$ | $R^4$ | $R^5$ | $R^6$ | y | n | Z | Q | $R^7$ | Salzform ($H_2O$-Gehalt in mol/mol) | Fp in °C |
|---|---|---|---|---|---|---|---|---|---|---|---|---|---|
| 66 | $CH_3$ | 7-Cl | 9-$CH_3$ | H | H | H | O | 2 | bi | N | 4-$CH_3$-pyrid-2- | BA | 251 |
| 67 | $CH_3$ | 7-OH | H | $CH_3$ | $CH_3$ | H | O | 2 | CO | N | 4-$CH_3$-pyrid-2- | BA | 204-206 |
| 68 | $CH_3$ | 7-$OCH_3$ | H | H | H | H | O | 2 | CO | N | 4-$CH_3$-pyrid-2- | BA (0,6) | 129-133 |
| 69 | $CH_3$ | 7-OH | H | H | H | H | O | 2 | CO | N | 4-$CH_3$-pyrid-2- | BA (1,5) | 148-150 |
| 70 | $CH_3$ | 7-$CH_3$ | 9-$CH_3$ | H | H | H | O | 2 | bi | N | 4-$CH_3$-pyrid-2- | BA (2,2) | 174-176 |
| 71 | $CH_3$ | 7-OH | H | $CH_3$ | $CH_3$ | H | O | 2 | CO | N | pyrid-2- | BA (1,4) | 130-132 |
| 72 | $CH_3$ | 7-OH | H | $OCH_3$ | $CH_3$ | H | O | 2 | CO | N | pyrid-2- | BA (0,5) | 135 |
| 73 | $CH_3$ | 7-OH | H | $CH_3$ | $CH_3$ | H | O | 3 | bi | N | 4-$CH_3$-pyrid-2- | BA (0,7) | 117-119 |
| 74 | $CH_3$ | 7-OH | H | $CH_3$ | $CH_3$ | H | O | 3 | bi | N | 3-$CH_3$-pyrid-2- | BA (1,1) | 118-120 |
| 75 | $CH_3$ | 7-$OCH_2$ \| phen | H | $CH_3$ | $CH_3$ | H | O | 2 | CO | N | pyrid-2- | BA (0,3) | 107-108 |

pyrid-2 = pyridin-2-yl, bi = Bindung, phen = phenyl, opyrim = 4-oxo-3H-pyrimidin-2-yl, BA = Base, HCl = Hydrochlorid, Z = Zersetzung

Beispiel I:

2,3-Dihydro-5-(4-hydroxyphenyl)-6-methyl-2-[2-(4-(4-methyl-pyridin-2-yl)-piperazin-1-yl)-ethyl]-pyrrolo[1,2,3-de]-1,4-benzoxazin enthaltende Tabletten.

Man stellte Tabletten in folgender Zusammensetzung pro Tablette her:

| | |
|---|---|
| 2,3-Dihydro-5-(4-hydroxyphenyl)-6-methyl-2-[2-(4-(4-methylpyridin-2-yl)-piperazin-1-yl)-ethyl]-pyrrolo[1,2,3-de]-1,4-benzoxazin | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

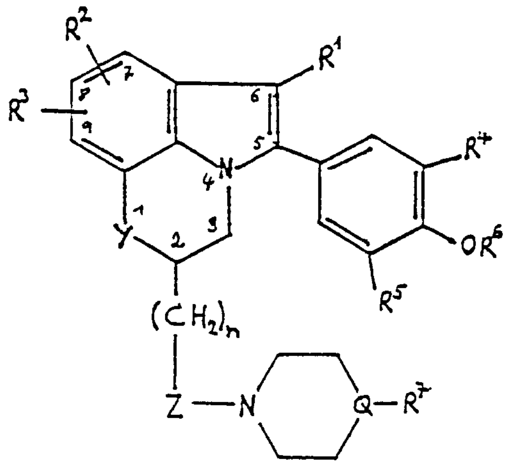

I

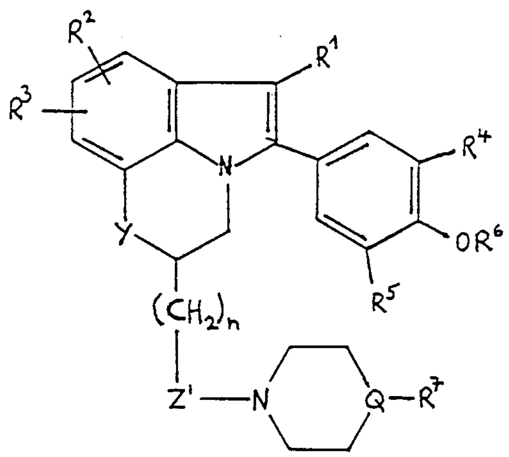

Ia

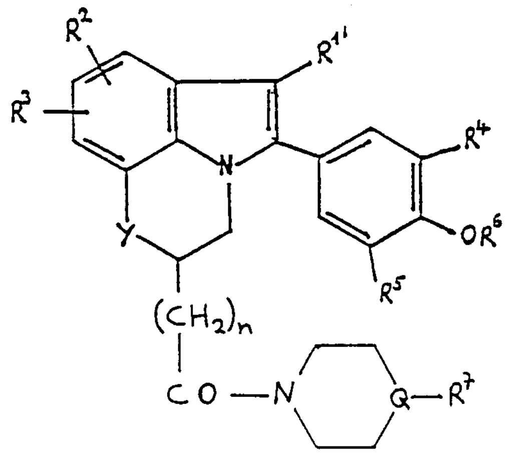

Ib

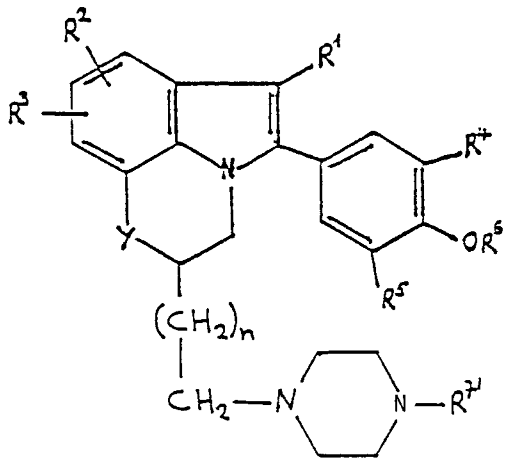

Ic

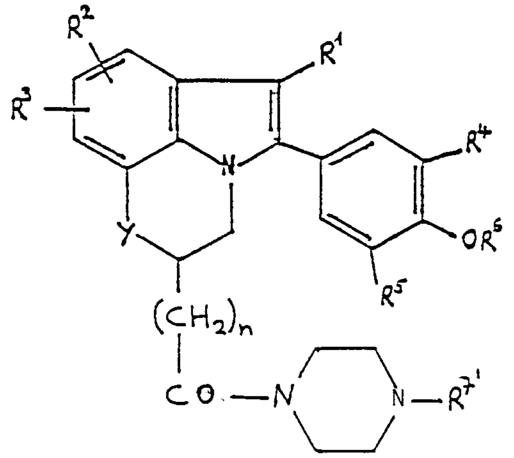

Id

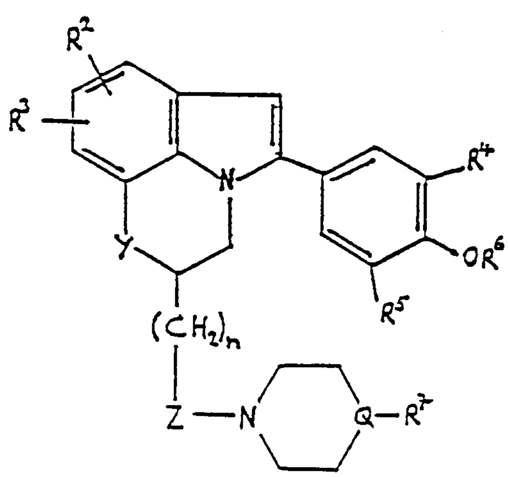

Ie

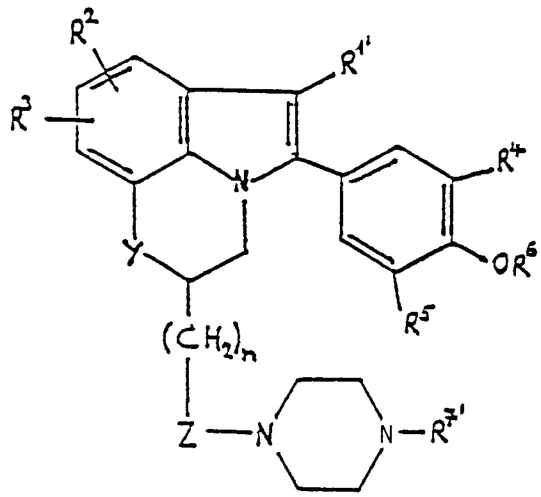

If

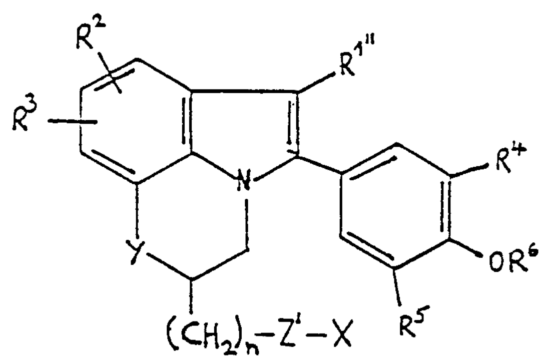

II

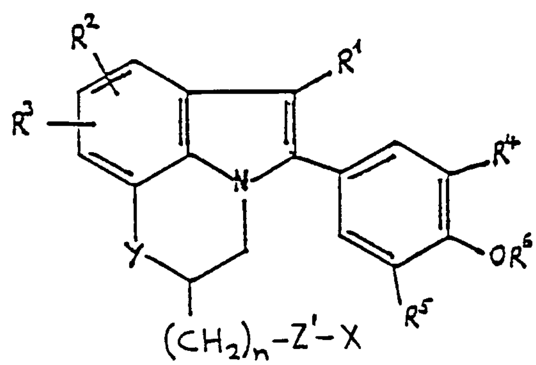

IIa

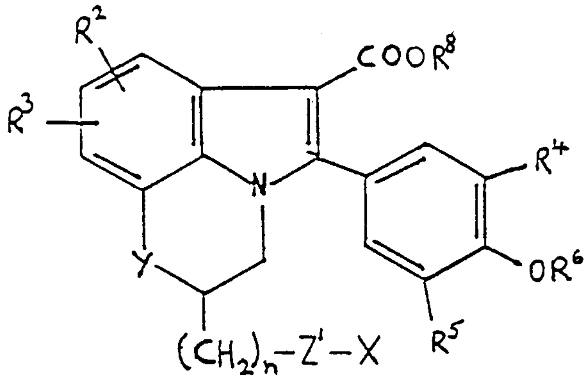

IIb

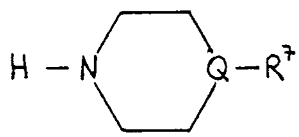

III

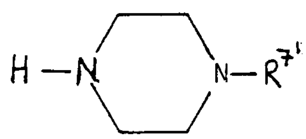

IIIa

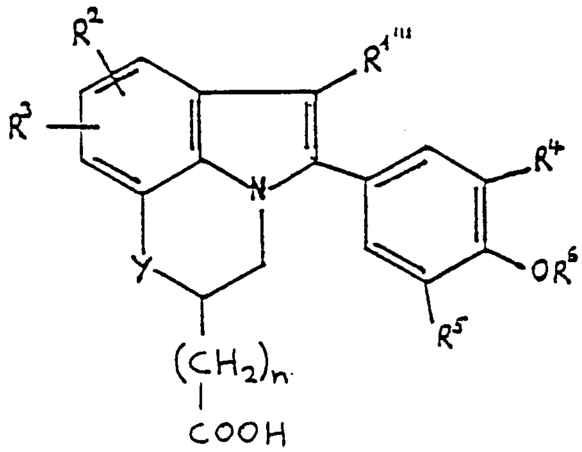

IV

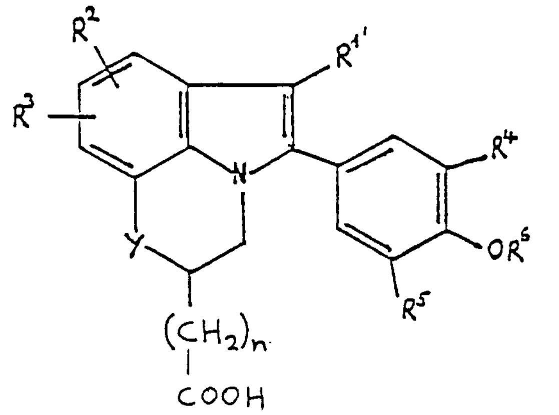

IVa

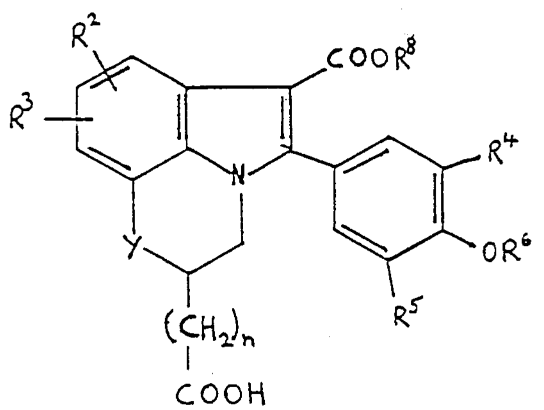

IVb

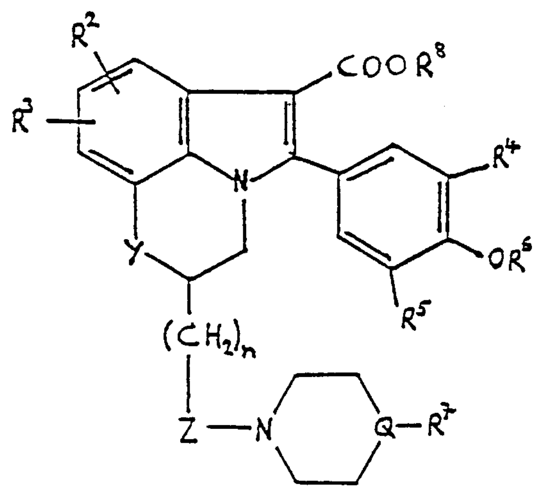

V

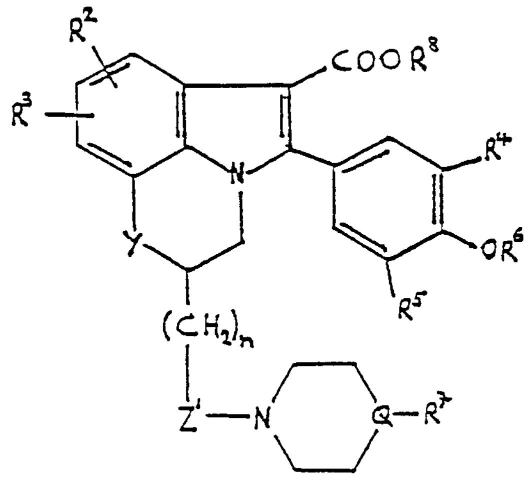

Va

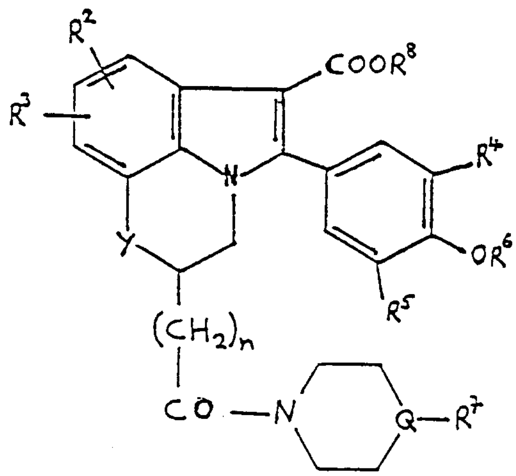

Vb

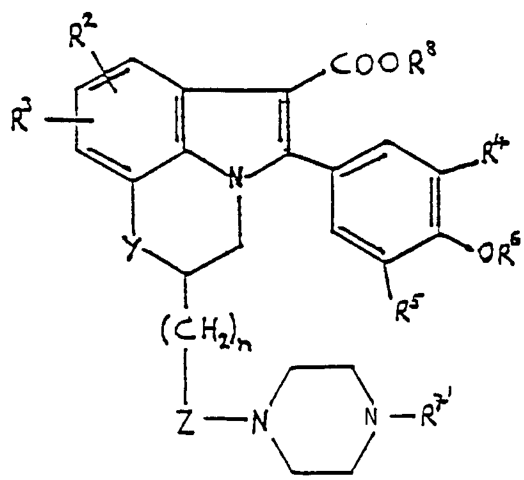

Vc

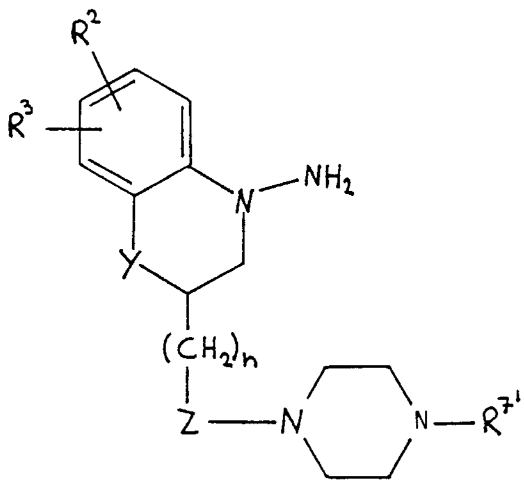

VI

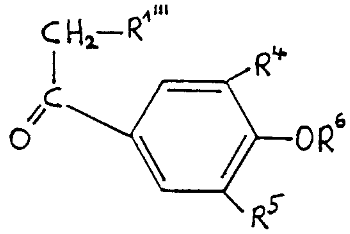

VII

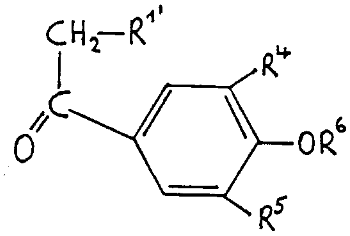

VIIa

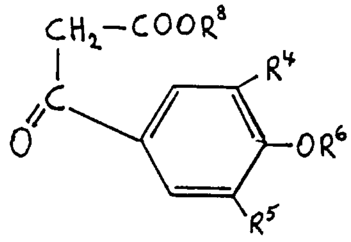

VIIb

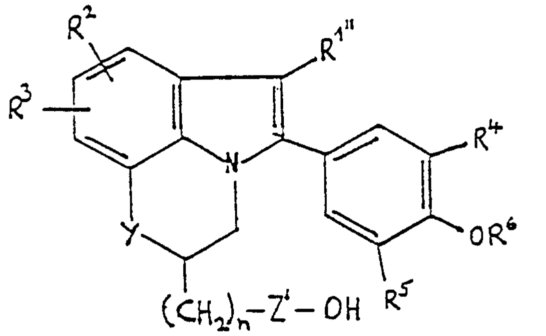

VIII

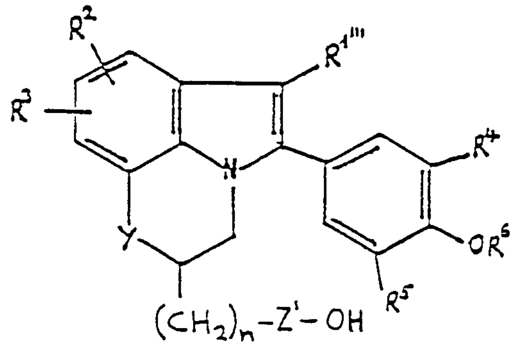

VIIIa

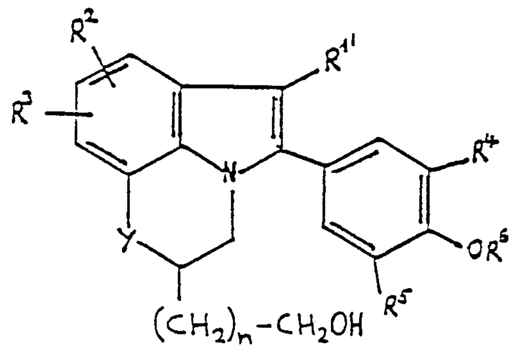

VIIIb

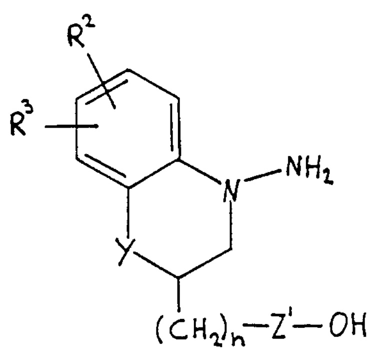

IX

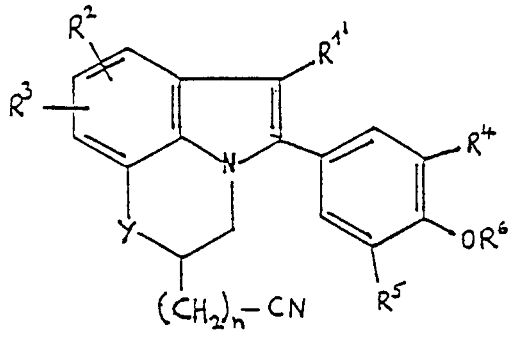

X

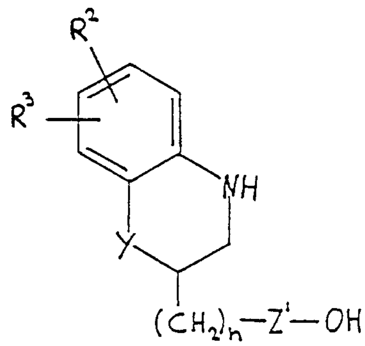

XI

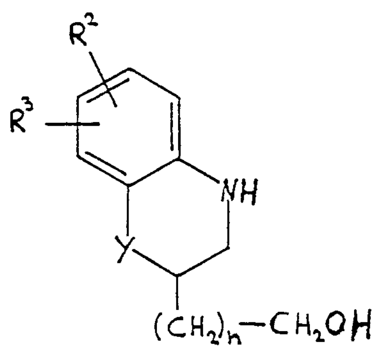

XIa

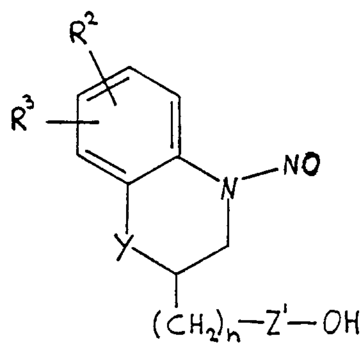

XII

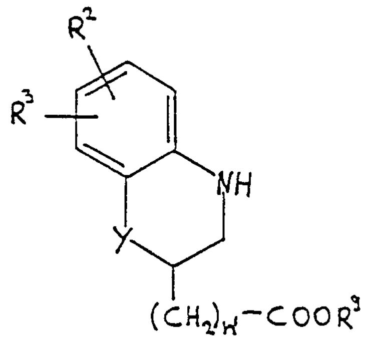

XIII

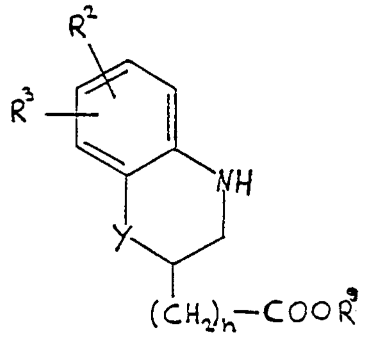

XIIIa

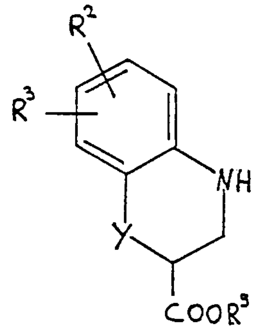

XIIIb

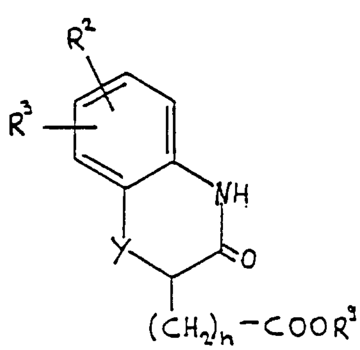

XIV

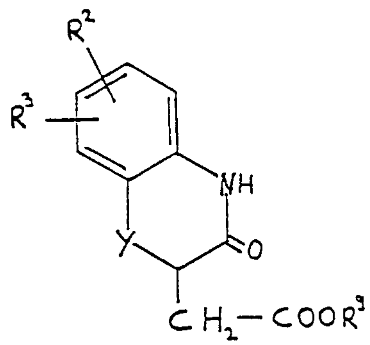

XIVa

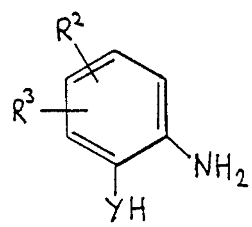

XV

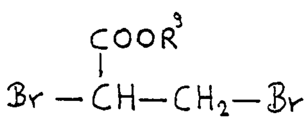

XVI

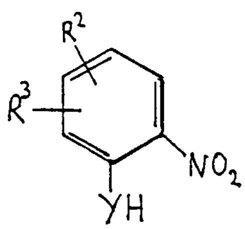

XVII

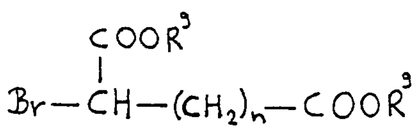

XVIII

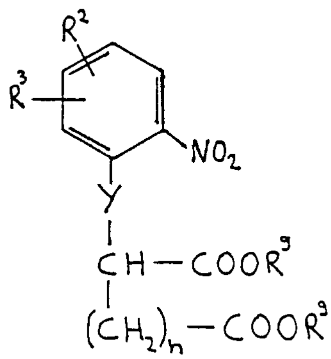

XIX

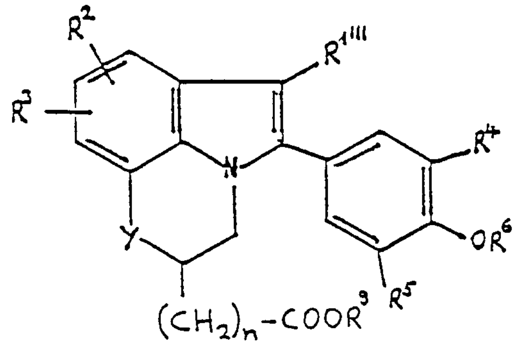

XX

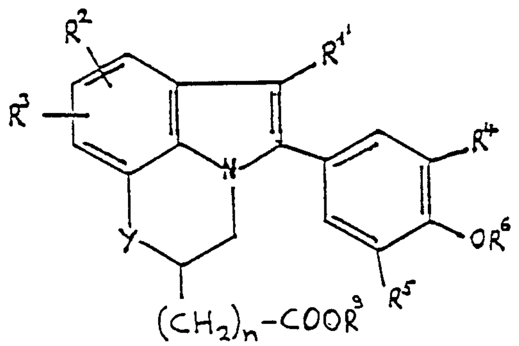

XXa

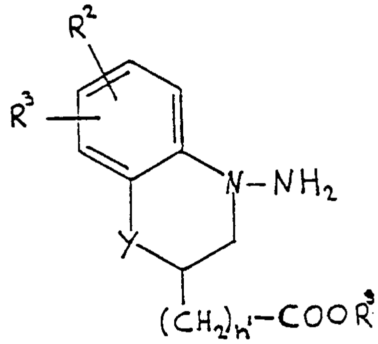

XXI

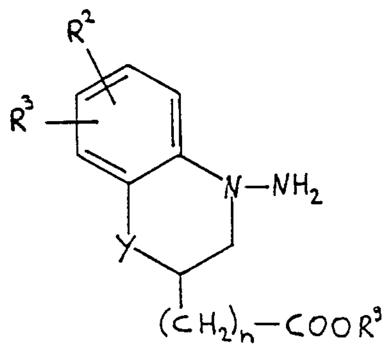

XXIa

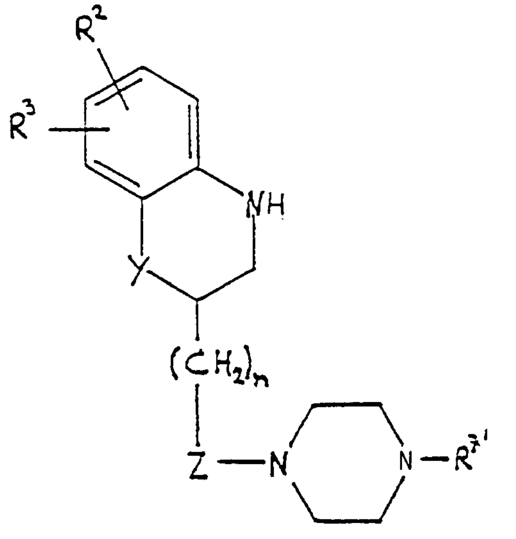

XXII

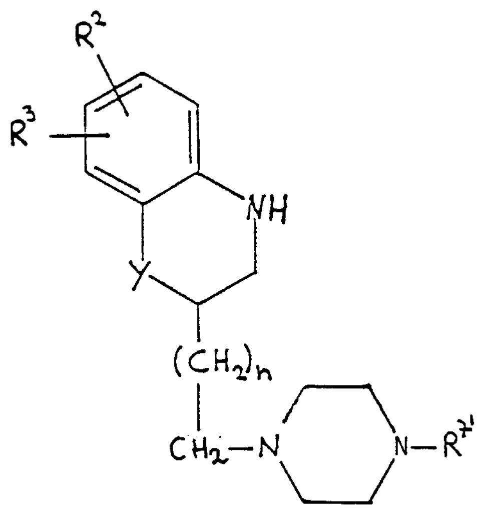

XXIIa

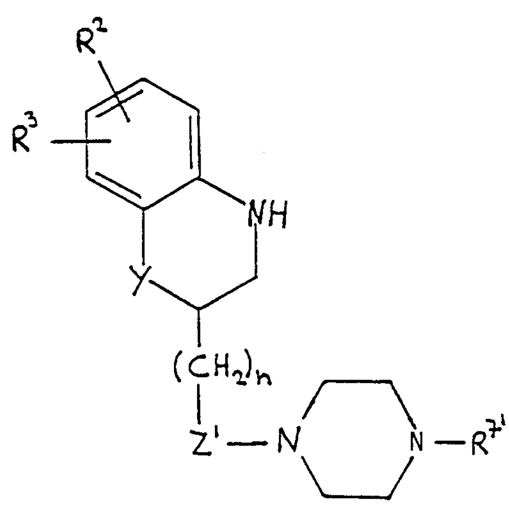

XXIIb

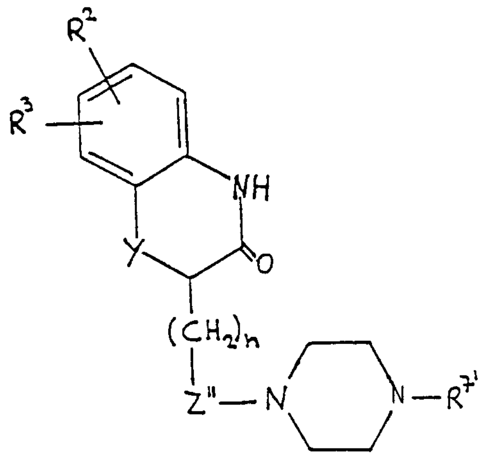

XXIII

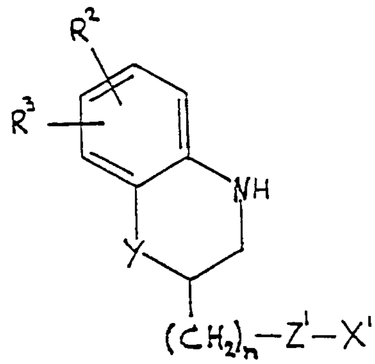

XXIV

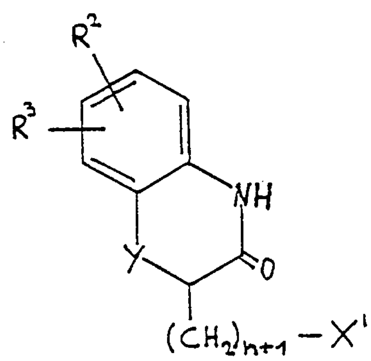

XXV

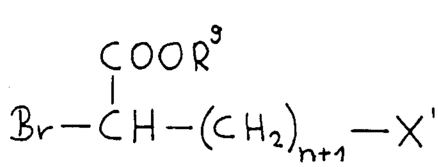

XXVI

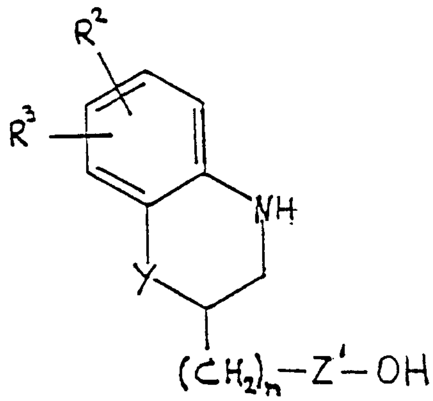

XXVII

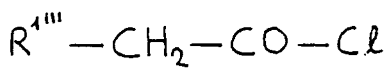

XXVIII

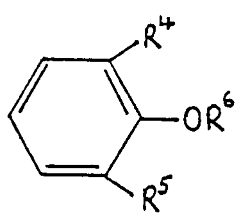

XXIX

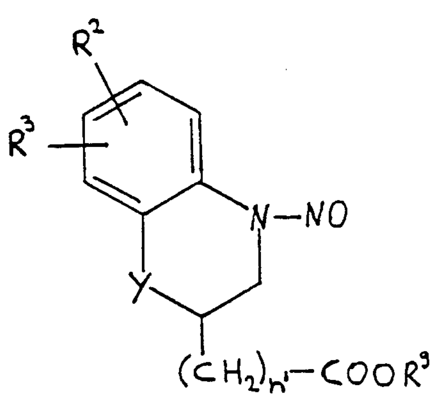

XXX

## Patentansprüche

**1.** Verbindungen der allgemeinen Formel I

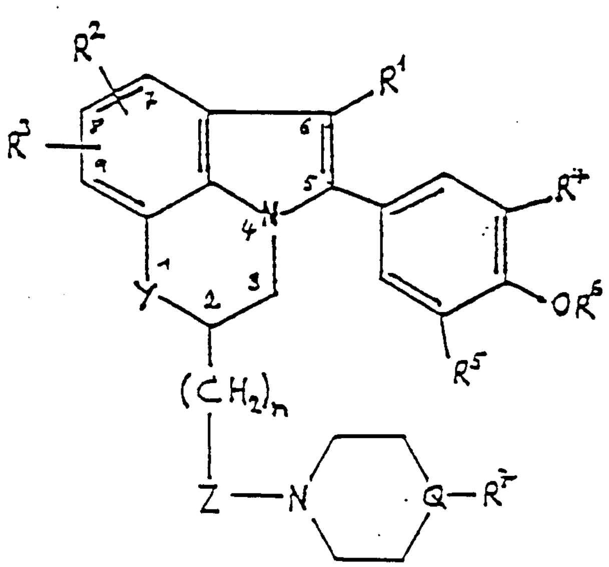

I

worin

$R^1$ Wasserstoff oder niederes Alkyl bedeutet,
$R^2$ Wasserstoff, Halogen, niederes Alkyl, niederes Alkoxy, Benzyloxy oder Hydroxy bedeutet,
$R^3$ Wasserstoff, Halogen, niederes Alkyl, niederes Alkoxy oder Hydroxy bedeutet,
$R^4$ Wasserstoff, niederes Alkyl, niederes Alkoxy oder Hydroxy bedeutet,
$R^5$ Wasserstoff, niederes Alkyl, niederes Alkoxy oder Hydroxy bedeutet,
$R^6$ Wasserstoff oder niederes Alkyl bedeutet,
Y Sauerstoff oder Schwefel bedeutet,
n für eine Zahl von 1 bis 3 steht,
Z eine Bindung, die CO-Gruppe oder die $CH_2$-Gruppe darstellt,
Q Stickstoff oder die CH-Gruppe bedeutet und
$R^7$ ,falls Q Stickstoff bedeutet, für einen gegebenenfalls durch niederes Alkyl oder Halogen substituierten
Pyridyl- oder Phenylrest steht, oder, falls Q die CH-Gruppe bedeutet, für die N-Methyl-N-(4-oxo-3H-pyrimidin-2-yl)-aminogruppesteht,

und deren physiologisch verträgliche Säureadditionssalze.

2. Verbindungen gemäß Anspruch 1, dadurch gekennzeichnet, daß $R^2$ Wasserstoff, Halogen, niederes Alkyl, niederes Alkoxy oder Hydroxy bedeutet.

3. Verbindungen gemäß einem der vorstehenden Ansprüche, dadurch gekennzeichnet, daß $R^6$ Wasserstoff bedeutet.

4. Verbindungen gemäß einem der vorstehenden Ansprüche, dadurch gekennzeichnet, daß Y Sauerstoff bedeutet.

5. Verbindungen gemäß einem der vorstehenden Ansprüche, dadurch gekennzeichnet, daß $R^1$ niederes Alkyl, insbesondere Methyl bedeutet.

6. Verbindungen gemäß einem der vorstehenden Ansprüche, dadurch gekennzeichnet, daß Q Stickstoff bedeutet und $R^7$ einen gegebenenfalls durch niederes Alkyl oder Halogen substituierten Pyridylrest bedeutet.

7. Verbindungen gemäß Anspruch 6, dadurch gekennzeichnet, daß $R^7$ den 4-Methylpyrid-2-ylrest darstellt.

8. Arzneimittel enthaltend eine pharmakologisch wirksame Menge einer Verbindung gemäß Anspruch 1 und übliche pharmazeutische Hilfs- und/oder Trägerstoffe.

**9.** Verfahren zur Herstellung von Verbindungen der allgemeinen Formel I

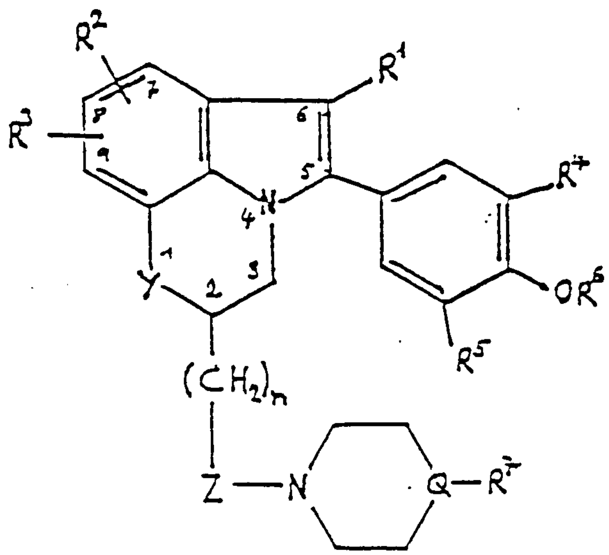

I

worin

- $R^1$ Wasserstoff oder niederes Alkyl bedeutet,
- $R^2$ Wasserstoff, Halogen, niederes Alkyl, niederes Alkoxy, Benzyloxy oder Hydroxy bedeutet,
- $R^3$ Wasserstoff, Halogen, niederes Alkyl, niederes Alkoxy oder Hydroxy bedeutet,
- $R^4$ Wasserstoff, niederes Alkyl, niederes Alkoxy oder Hydroxy bedeutet,
- $R^5$ Wasserstoff, niederes Alkyl, niederes Alkoxy oder Hydroxy bedeutet,
- $R^6$ Wasserstoff oder niederes Alkyl bedeutet,
- Y Sauerstoff oder Schwefel bedeutet,
- n für eine Zahl von 1 bis 3 steht,
- Z eine Bindung, die CO-Gruppe oder die $CH_2$-Gruppe darstellt,
- Q Stickstoff oder die CH-Gruppe bedeutet und
- $R^7$ ,falls Q Stickstoff bedeutet, für einen gegebenenfalls durch niederes Alkyl oder Halogen substituierten Pyridyl- oder Phenylrest steht, oder, falls Q die CH-Gruppe bedeutet, für die N-Methyl-N-(4-oxo-3H-pyrimidin-2-yl)-aminogruppe steht,

und deren physiologisch verträglichen Säureadditionssalzen, dadurch gekennzeichnet, daß man

a) zur Herstellung von Verbindungen der allgemeinen Formel Ia

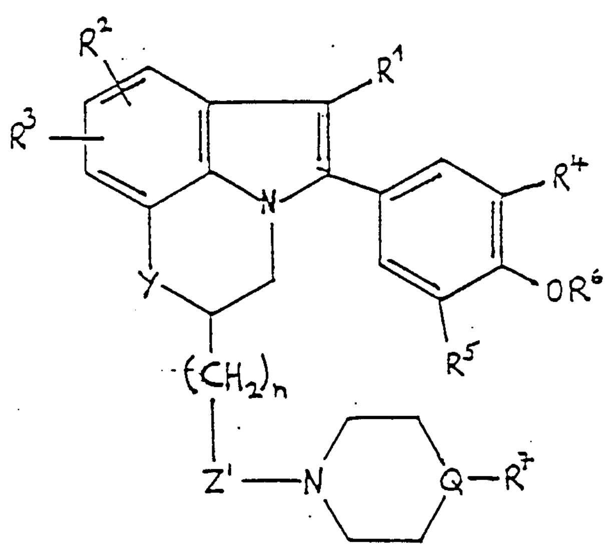

Ia

worin $R^1$, $R^2$, $R^3$, $R^4$, $R^5$, $R^6$, n, Y, Q und $R^7$ obige Bedeutung besitzen und Z' eine Bindung oder die $CH_2$-Gruppe darstellt, Verbindungen der allgemeinen Formel IIa

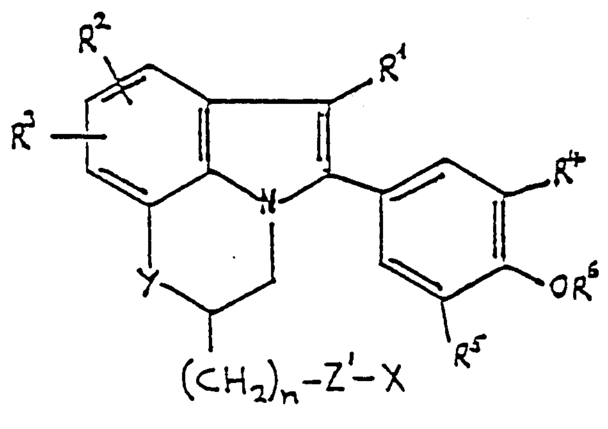

IIa

worin $R^1$, $R^2$, $R^3$, $R^4$, $R^5$, $R^6$, n, Z' und Y obige Bedeutung besitzen, und X für eine aminolytisch abspaltbare Fluchtgruppe steht,
mit Verbindungen der allgemeinen Formel III

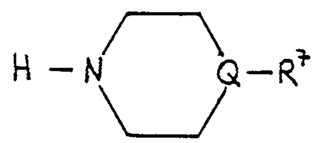

III

worin Q und $R^7$ obige Bedeutung besitzen, umsetzt oder
b) zur Herstellung von Verbindungen der allgemeinen Formel Ib

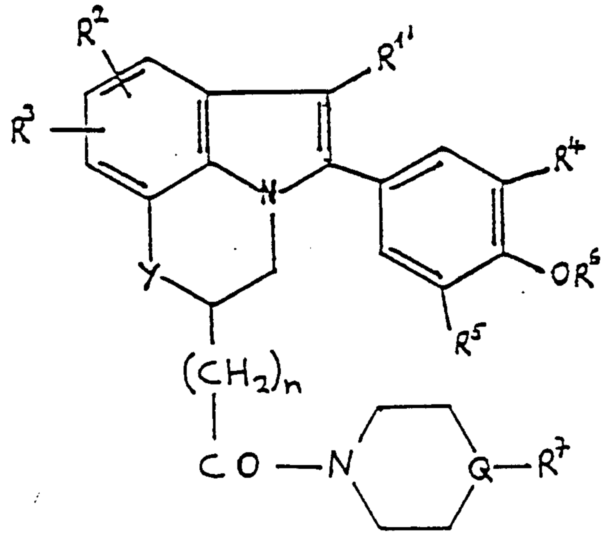

Ib

worin $R^2$, $R^3$, $R^4$, $R^5$, $R^6$, Y, n, Q und $R^7$ obige Bedeutung besitzen und $R^{1'}$ niederes Alkyl bedeutet, Verbindungen der allgemeinen Formel IVa

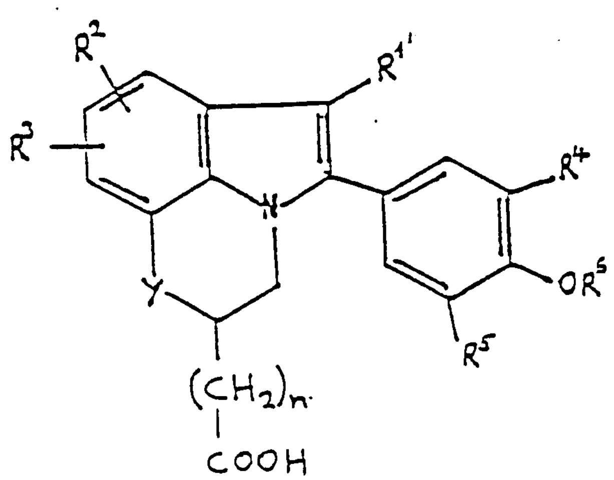

IVa

worin $R^{1'}$, $R^2$, $R^3$, $R^4$, $R^5$, $R^6$, Y und n obige Bedeutung besitzen, mit Verbindungen der Formel III umsetzt oder

c) zur Herstellung von Verbindungen der allgemeinen Formel Ic

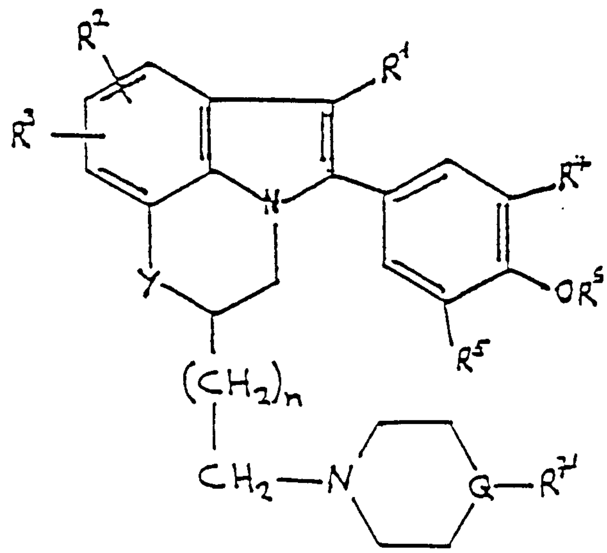

Ic

worin $R^1$, $R^2$, $R^3$, $R^4$, $R^5$, $R^6$, Y und n obige Bedeutung besitzen und $R^{7'}$ für einen gegebenenfalls durch niederes Alkyl oder Halogen substituierten Pyridyl- oder Phenylrest steht, Verbindungen der allgemeinen Formel Id

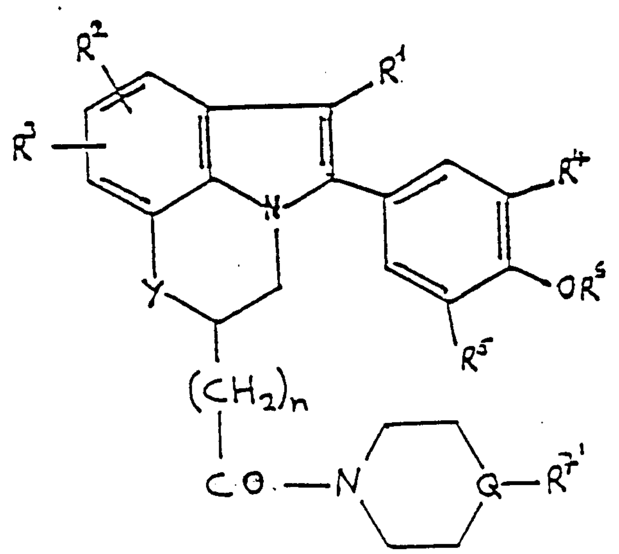

Id

worin $R^1$, $R^2$, $R^3$, $R^4$, $R^5$, $R^6$, Y, n und $R^{7'}$ obige Bedeutung besitzen, reduziert, oder
d) zur Herstellung von Verbindungen der allgemeinen Formel Ie

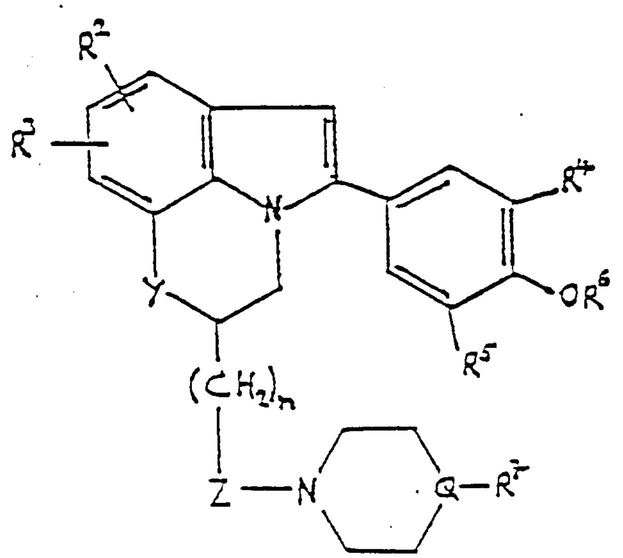

Ie

worin $R^2$, $R^3$, $R^4$, $R^5$, $R^6$, Y, n, Z, Q und $R^7$ obige Bedeutung besitzen, aus Verbindungen der allgemeinen Formel V

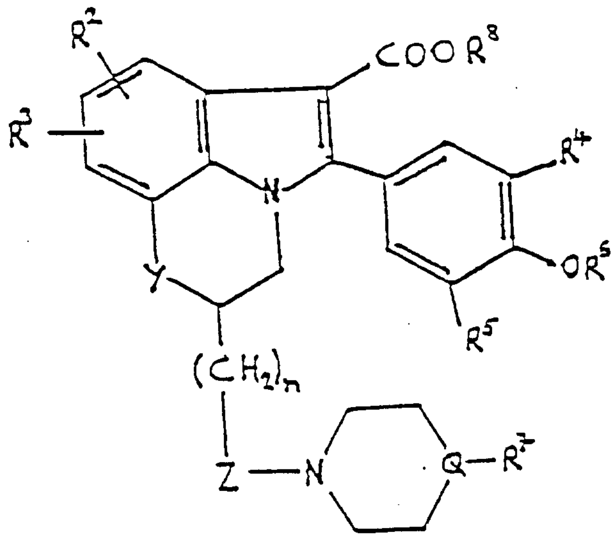

V

worin $R^2$, $R^3$, $R^4$, $R^5$, $R^6$, Y, n, Z, Q und $R^7$ obige Bedeutung besitzen und $R^8$ niederes Alkyl bedeutet, die $COOR^8$-Gruppe abspaltet, oder

e) zur Herstellung von Verbindungen der allgemeinen Formel If

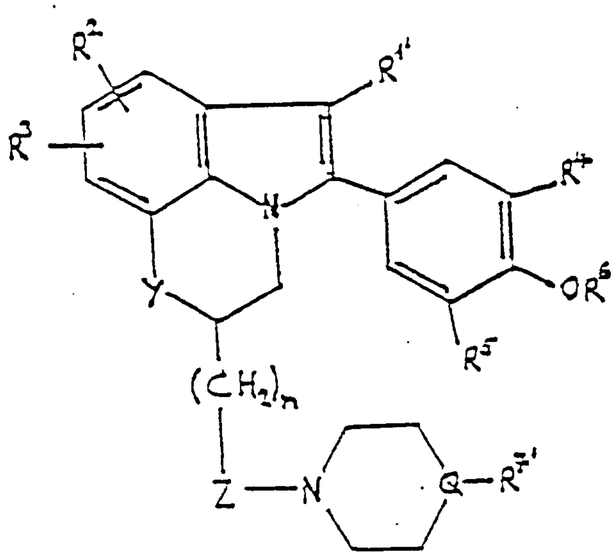

If

worin $R^{1'}$, $R^2$, $R^3$, $R^4$, $R^5$, $R^6$, Y, n, Z und $R^{7'}$ obige Bedeutung besitzen, Verbindungen der allgemeinen Formel VI

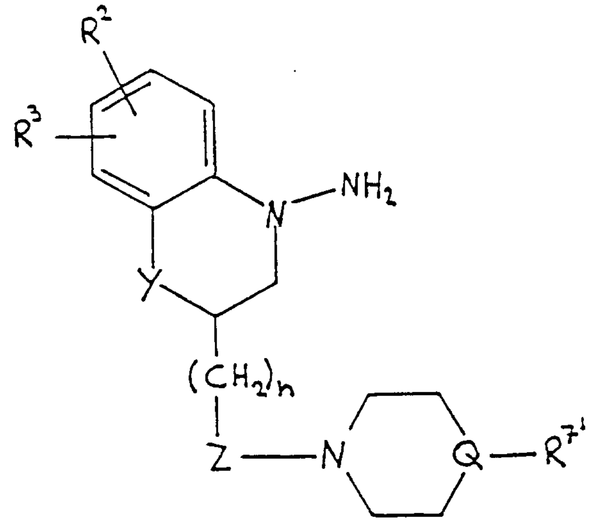

VI

worin $R^2$, $R^3$, Y, n, Z und $R^{7'}$ obige Bedeutung besitzen, umsetzt mit Verbindungen der allgemeinen Formel VIIa

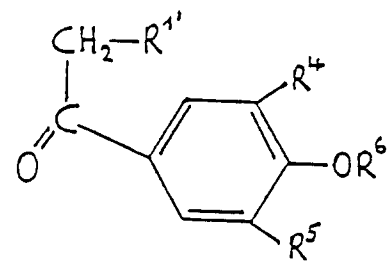

VIIa

worin $R^{1'}$, $R^4$, $R^5$ und $R^6$ obige Bedeutung besitzen,
und in den erhaltenen Verbindungen der Formel I gewünschtenfalls Methoxysubstituenten $R^2$, $R^3$, $R^4$, $R^5$ und/oder $OR^6$ in Hydroxy überführt und/oder einen Benzyloxysubstituenten $R^2$ in Hydroxy überführt, und die erhaltenen Verbindungen der Formel I gewünschtenfalls in ihre Säureadditionssalze überführt oder die Säureadditionssalze in die freien Verbindungen der Formel I überführt.

**10.** Verbindungen der allgemeinen Formel II

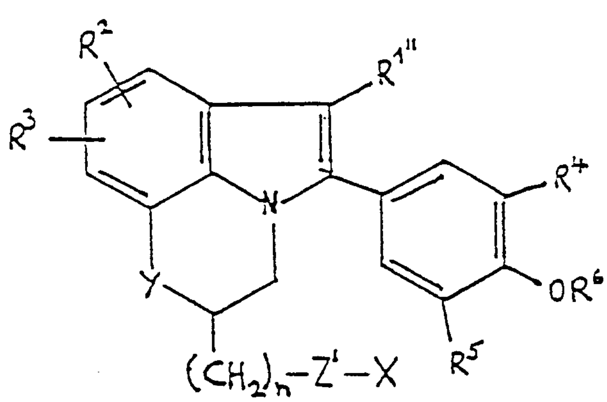

II

worin

$R^{1''}$ Wasserstoff, niederes Alkyl oder eine Alkoxycarbonylgruppe $COOR^8$, worin $R^8$ niederes Alkyl ist, bedeutet,

$R^2$ Wasserstoff, Halogen, niederes Alkyl, niederes Alkoxy, Benzyloxy oder Hydroxy bedeutet,
$R^3$ Wasserstoff, Halogen, niederes Alkyl, niederes Alkoxy oder Hydroxy bedeutet,
$R^4$ Wasserstoff, niederes Alkyl, niederes Alkoxy oder Hydroxy bedeutet,
$R^5$ Wasserstoff, niederes Alkyl, niederes Alkoxy oder Hydroxy bedeutet,
$R^6$ Wasserstoff oder niederes Alkyl bedeutet,
Y Sauerstoff oder Schwefel bedeutet,
n für eine Zahl von 1 bis 3 steht,
Z' eine Bindung oder $CH_2$-Gruppe darstellt, und
X eine aminolytisch abspaltbare Fluchtgruppe, vorzugsweise Halogen oder einen Acyloxyrest O-E, worin E einen niederen Alkanoylrest oder einen organischen Sulfonsäurerest bedeutet, darstellt.

**11.** Verbindungen der allgemeinen Formel VIII

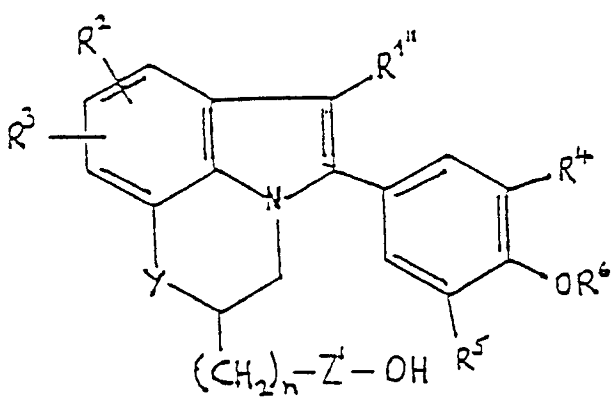

VIII

worin
$R^{1'''}$ Wasserstoff, niederes Alkyl oder eine Alkoxycarbonylgruppe $COOR^8$, worin $R^8$ niederes Alkyl ist, bedeutet,
$R^2$ Wasserstoff, Halogen, niederes Alkyl, niederes Alkoxy, Benzyloxy oder Hydroxy bedeutet,
$R^3$ Wasserstoff, Halogen, niederes Alkyl, niederes Alkoxy oder Hydroxy bedeutet,
$R^4$ Wasserstoff, niederes Alkyl, niederes Alkoxy oder Hydroxy bedeutet,
$R^5$ Wasserstoff, niederes Alkyl, niederes Alkoxy oder Hydroxy bedeutet,
$R^6$ Wasserstoff oder niederes Alkyl bedeutet,
Y Sauerstoff oder Schwefel bedeutet,
n für eine Zahl von 1 bis 3 steht, und
Z' eine Bindung, die CO-Gruppe oder die $CH_2$-Gruppe darstellt.

**12.** Verbindungen der allgemeinen Formel IV

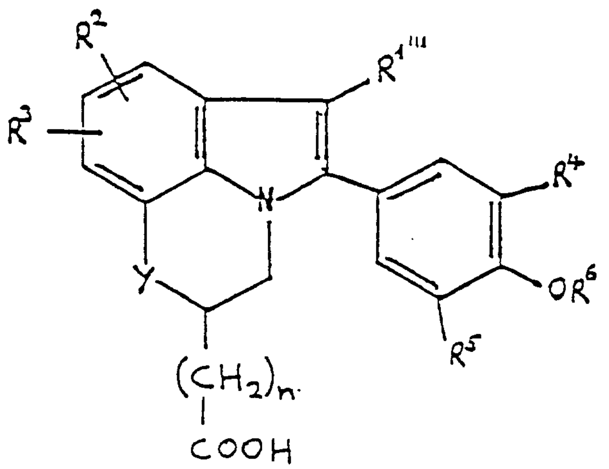

IV

worin

- $R^{1'''}$ niederes Alkyl oder eine Alkoxycarbonylgruppe $COOR^8$, worin $R^8$ niederes Alkyl ist, bedeutet,
- $R^2$ Wasserstoff, Halogen, niederes Alkyl, niederes Alkoxy, Benzyloxy oder Hydroxy bedeutet,
- $R^3$ Wasserstoff, Halogen, niederes Alkyl, niederes Alkoxy oder Hydroxy bedeutet,
- $R^4$ Wasserstoff, niederes Alkyl, niederes Alkoxy oder Hydroxy bedeutet,
- $R^5$ Wasserstoff, niederes Alkyl, niederes Alkoxy oder Hydroxy bedeutet,
- $R^6$ Wasserstoff oder niederes Alkyl bedeutet,
- Y Sauerstoff oder Schwefel bedeutet, und
- n für eine Zahl von 1 bis 3 steht.

**13.** Verbindungen der allgemeinen Formel V

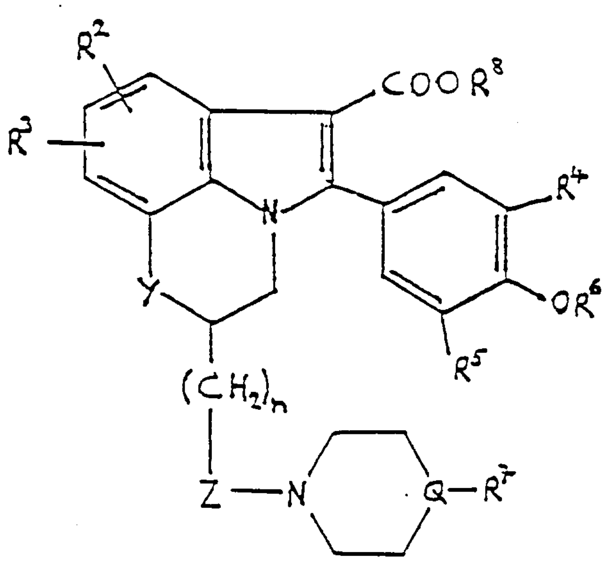

V

worin

- $R^8$ niederes Alkyl bedeutet,
- $R^2$ Wasserstoff, Halogen, niederes Alkyl, niederes Alkoxy, Benzyloxy oder Hydroxy bedeutet,
- $R^3$ Wasserstoff, Halogen, niederes Alkyl, niederes Alkoxy oder Hydroxy bedeutet,
- $R^4$ Wasserstoff, niederes Alkyl, niederes Alkoxy oder Hydroxy bedeutet,
- $R^5$ Wasserstoff, niederes Alkyl, niederes Alkoxy oder Hydroxy bedeutet,
- $R^6$ Wasserstoff oder niederes Alkyl bedeutet,

Y Sauerstoff oder Schwefel bedeutet,
n für eine Zahl von 1 bis 3 steht,
Z eine Bindung, die CO-Gruppe oder die $CH_2$-Gruppe darstellt,
Q Stickstoff oder die CH-Gruppe bedeutet und
$R^7$ ,falls Q Stickstoff bedeutet, für einen gegebenenfalls durch niederes Alkyl oder Halogen substituierten Pyridyl- oder Phenylrest steht, oder, falls Q die CH Gruppe bedeutet, für die N-Methyl-N-(4-oxo-3H-pyrimidin-2-yl)-aminogruppe steht.

**14.** Verbindungen der allgemeinen Formel VI

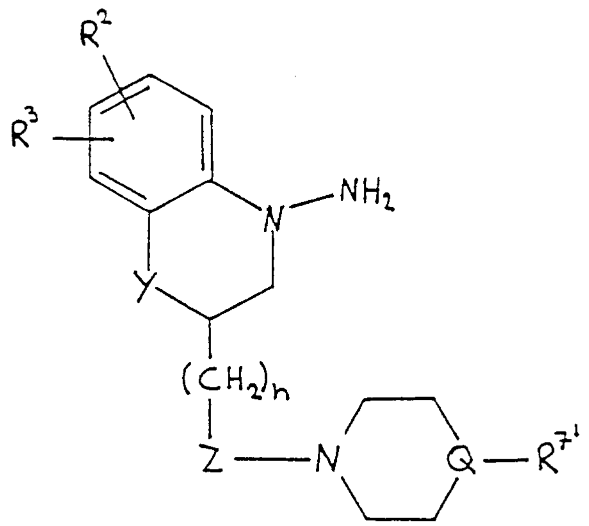

VI

worin
$R^2$ Wasserstoff, Halogen, niederes Alkyl, niederes Alkoxy, Benzyloxy oder Hydroxy bedeutet,
$R^3$ Wasserstoff, Halogen, niederes Alkyl, niederes Alkoxy oder Hydroxy bedeutet,
Y Sauerstoff oder Schwefel bedeutet,
n für eine Zahl von 1 bis 3 steht,
Z eine Bindung, die CO-Gruppe oder die $CH_2$-Gruppe darstellt, und
$R^{7'}$ für einen gegebenenfalls durch niederes Alkyl oder Halogen substituierten Pyridyl- oder Phenylrest steht.

Europäisches Patentamt

# EUROPÄISCHER RECHERCHENBERICHT

Nummer der Anmeldung
EP 94 10 1885

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X<br>A | WO-A-90 07505 (STERLING DRUG)<br>* Anspruch 22 *<br>* Ansprüche 1,34 *<br>--- | 14<br><br>1,8 | C07D498/06<br>C07D513/06<br>C07D279/16<br>C07D265/36<br>A61K31/535<br>A61K31/54<br>//(C07D498/06, 265:00, 209:00), (C07D513/06, 279:00,209:00) |
| A | CHEMICAL AND PHARMACEUTICAL BULLETIN.<br>Bd. 34, Nr. 1 , 1986 , TOKYO JP<br>Seiten 130 - 139<br>Y. MASUOKA ET AL 'Synthesesof 3,4-dihydro-2H-1,4-benzoxazine-2-acetates and related compounds'<br>* Seite 136; Beispiele p,q; Tabelle I *<br>--- | 1,8 | |
| A | FR-A-2 567 126 (SYNTHELABO)<br>* Ansprüche 1,4 *<br>--- | 1,8 | |
| A | CHEMICAL ABSTRACTS, vol. 103, no. 11, 16. September 1985, Columbus, Ohio, US;<br>abstract no. 87826r,<br>G. TRAPANI ET AL 'Synthesis and antiinflammatory activities of some 1,4-benzothiazine derivatives'<br>Seite 605 ;<br>* Zusammenfassung *<br>& FARMACO, ED. SCI.<br>Bd. 40, Nr. 5 , 1985<br>Seiten 369 - 376<br>--- | 1,8 | **RECHERCHIERTE SACHGEBIETE (Int.Cl.5)**<br>C07D<br>A61K |
| P,A | EP-A-0 529 452 (KALI-CHEMIE PHARMA)<br><br>* Ansprüche 1,9,11-14 *<br>----- | 1,8,<br>10-14 | |

Der vorliegende Recherchenbericht wurde für alle Patentansprüche erstellt

| Recherchenort | Abschlußdatum der Recherche | Prüfer |
|---|---|---|
| DEN HAAG | 26. Mai 1994 | Voyiazoglou, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument